(19) Europäisches Patentamt
European Patent Office
Office européen des brevets

(11) Numéro de publication: **0 169 111**
**B1**

# (12) FASCICULE DE BREVET EUROPEEN

(45) Date de publication du fascicule du brevet: **08.02.89**

(51) Int. Cl.⁴: **C 07 K 15/00**, A 61 K 39/395, A 61 K 47/00

(21) Numéro de dépôt: **85401199.6**

(22) Date de dépôt: **18.06.85**

(54) **Nouveaux conjugués cytotoxiques utilisables en thérapeutique et procédé d'obtention.**

**Demande divisionnaire 87202356 déposée le 30.11.87.**

(30) Priorité: **20.06.84 FR 8409703**

(43) Date de publication de la demande:
**22.01.86 Bulletin 86/4**

(45) Mention de la délivrance du brevet:
**08.02.89 Bulletin 89/6**

(84) Etats contractants désignés:
**AT BE CH DE FR GB IT LI LU NL SE**

(56) Documents cités:
**EP-A- 0 023 401**
**EP-A- 0 044 167**
**EP-A- 0 080 401**
**FR-A- 2 437 213**
**FR-A- 2 466 252**

**Immunological Review, 62-1982-185-216-F.K. Fransen et al.**
**J.Biochem -91-1982-1583-1591- Masuho et al.**

(73) Titulaire: **SANOFI, 40, Avenue George V, F-75008 Paris (FR)**

(72) Inventeur: **Jansen, Franz K., Chemin des Fresquets Assas, F-34160 Castries (FR)**
Inventeur: **Gros, Pierre, 18 rue des Muriers, F-34100 Montpellier (FR)**

(74) Mandataire: **Gillard, Marie-Louise et al, Cabinet Beau de Loménie 55, Rue d'Amsterdam, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux conjugués cytotoxiques, un procédé pour leur préparation et des compositions pharmaceutiques à action cytotoxique.

Le brevet US 4 340 535 décrit la préparation de produits anticancéreux, dits conjugués ou encore immunotoxines, obtenus par couplage par liaison covalente de la chaîne A de la ricine à une structure protéique, telle qu'un anticorps, une immunoglobuline ou un fragment d'immunoclobuline, capable de reconnaître sélectivement un antigène donné à la surface des cellules porteuses de cet antigène que l'on veut atteindre, telles que les cellules cancéreuses. La propriété principale de ces conjugués est d'être des agents cytotoxiques spécifiques des cellules cibles visées.

L'utilisation d'anticorps dirigés, soit contre des haptènes, soit contre des antigènes de différentiation, soit contre des antigènes associés aux cellules cancéreuses avait déjà permis d'obtenir des conjugués présentant une spécificité remarquable vis-à-vis des cellules cibles et un pouvoir cytotoxique très élevé vis-à-vis de ces mêmes cellules, comme cela apparaît dans le brevet ci-dessus.

Les conjugués possédant ces propriétés étaient toujours des molécules mixtes artificielles dans lesquelles la chaîne A de la ricine était associée par une liaison covalente de type disulfure à un anticorps, une immunoglobuline ou un fragment d'immunoglobuline capable de reconnaître sélectivement un antigène porté par les cellules cibles visées.

Les conjugués décrits dans le brevet ci-dessus sont caractérisés par la présence en même temps des trois propriétés suivantes:

- la liaison covalente entre la chaîne A de la ricine et l'anticorps contient un radical disulfure,
- l'un des atomes de soufre constituant la liaison disulfure est toujours l'atome de soufre appartenant au résidu de cystéine en position 257 de la chaîne A de ricine,
- le bras de liaison réunissant la chaîne A de ricine et l'anticorps est fixé sur ce dernier au niveau de groupements NH2 latéraux ou terminaux d'une chaîne peptidique.

Il a été maintenant trouvé qu'il n'est pas nécessaire que les trois conditions indiquées ci-dessus soient simultanément satisfaites pour que l'on puisse obtenir des conjugués possédant à un niveau satisfaisant la propriété de cytotoxicité sélective qui caractérise cette classe de produits anticancéreux.

La présente invention a pour objet des produits, appartenant à la classe des Immunotoxines, obtenus par couplage covalent entre, d'une part la chaîne A de ricine telle quelle ou convenablement modifiée (ci-après désignée par le symbole A) et, d'autre part un anticorps ou fragment d'anticorps, utilisé sous sa forme naturelle ou correctement modifié (ci-après désigné par le symbole P), possédant la capacité de reconnaître sélectivement un antigène porté par les cellules cibles visées. Le couplage entre les 2 protéines est réalisé par l'intermédiaire d'une liaison disulfure.

Ainsi, la présente invention concerne une immunotoxine de formule statistique

$$P'\text{–}W'\text{–}A' \qquad II$$

dans laquelle P' représente le radical d'une protéine qui est un anticorps ou un fragment d'anticorps tel quel ou chimiquement convenablement modifié, privé d'au moins l'une de ses fonctions propres et dans lequel les autres groupes fonctionnels sont éventuellement bloqués, A' représente le radical d'une protéine qui est la sous-unité A de la ricine telle quelle ou chimiquement convenablement modifiée privée d'au moins une de ses fonctions propres et dans laquelle les autres groupes fonctionnels sont éventuellement bloqués et

W' représente une structure covalente choisie parmi:

(a) un groupement de formule

$$-Z''\text{–}Y\text{–}E\text{–}S\text{–}S\text{–}(E'\text{–}Y'\text{–}Z')n-$$

(b) un groupement de formule

$$-(NH\text{–}Y'\text{–}E')n\text{–}S\text{–}S\text{–}E\text{–}Y\text{–}Z'-$$

où

–Z' et Z'' représentent respectivement les fonctions propres des protéines A et P, choisies parmi l'atome d'oxygène provenant de l'hydroxyle de l'un des résidus de tyrosine; le groupe carbonyle provenant de l'un des carboxyles terminaux ou de l'un des carboxyles libres des acides aspartiques et/ou glutamiques de A et de P; Z'' représente aussi le groupe provenant de la structure dialdéhydique obtenue après oxydation de l'une des structures glucidiques de P par l'acide periodique; Z' représente aussi le groupe –NH– provenant de l'une des amines terminales de A ou de l'une des amines en position epsilon de l'un des résidus de lysine de A;

- Y et Y' représentent des groupes fonctionnels capables de se lier d'une façon covalente avec l'une quelconque des fonctions Z' et Z'' des protéines A et P;
- E et E' représentent des structures d'espacement inertes; et
- n représente zéro ou 1.

Les immunotoxines de la présente invention sont représentées par la formule II ci-dessus en forme simplifiée, mais il est entendu que la structure covalente bivalente –W– ou –W'– est liée à au moins une molécule P et au moins à une molécule A. Le nombre de liaisons avec les protéines P et A dépend du nombre des fonctions propres desdites protéines intervenant dans l'opération de couplage.

Par exemple, si une immunotixine est formée par couplage de la sous-unité A de la ricine native avec l'anticorps P (par exemple l'anticorps T101) par l'intermédiaire d'une structure covalente bivalente ayant un groupe disulfure dont un soufre est celui de la cystéine 257 de la chaîne A de la ricine et l'autre est lié aux oxygènes phénoliques des tyrosines de l'anticorps P par un groupe oxopropylique, elle aura la formule statistique

P'(O–CO–CH2–CH2–S–S–A')t

dans laquelle t représente le nombre des tyrosines de l'anticorps (par exemple de l'anticorps T101) intervenues dans le couplage.

L'immunotoxine ainsi obtenue correspond ainsi à un produit de formule II, où:

– P' est tel que défini ci-dessus, notamment le radical de l'anticorps T101 privé de t fonctions phénoliques de ses tyrosines;

– A' est tel que défini ci-dessus, notamment le radical de la chaîne A de la ricine privé de la fonction thiol de sa cystéine 257;

– W' est le groupement (a):

–Z''–Y–E–S–S–(E'–Y'–Z')n–

où Z'' est l'oxygène des hydroxyles phénoliques intervenus dans le couplage, Y est –CO–; E est la structure d'espacement inerte –CH2–CH2– et n est zéro.

Particulièrement préférées sont les immunotoxines formées par une ou plusieurs structures contenant la sous-unité A de la ricine et un seul anticorps P, représentées par la formule statistique

P'(W'–A')m III

dans laquelle P', W' et A' sont tels que définis ci-dessus et m représente le nombre de fonctions propres de la protéine P intervenues dans le couplage. Le nombre m varie de 0,3 à 12, de préférence de 0,5 à 10.

L'expression «m varie de 0,3 à 12, de préférence de 0,5 à 10» signifie que la valeur de m est statistique car dans la population des molécules d'anticorps le couplage ne se produit pas d'une façon homogène. Le nombre m peut donc ne pas être entier.

La valeur de m dépend notamment des anticorps utilisés, plus particulièrement de leur poids moléculaire.

Ainsi, si comme anticorps P de départ on utilise un fragment Fab ou Fab', la valeur de m pourra varier entre 0,3 et environ 2; si l'on utilise un fragment F(ab')2, m pourra varier entre 0,5 et environ 4; pour un anticorps du type IgG, la valeur de m sera entre 0,5 et 6; enfin, pour un anticorps IgM, la valeur de m pourra varier entre 1 et 12.

Il est cependant préférable que le dégré de substitution sur l'anticorps P soit tel qu'il conduise à une valeur de m non inférieure à 0,5 et non supérieure à 10.

Plus généralement, la structure II ci-dessus représente des formules statistiques écrites de façon simplifiée, comme il a été spécifié ci-dessus.

D'une façon analogue, les formules IV, V et VI ci-dessous sont également statistiques – lorsque, le cas échéant, n est 1 – car les réactifs de couplage sont préparés à partir de populations de protéines P1 et P2 qui ont toutes exactement les mêmes propriétés que celles prises en compte ci-dessus pour l'anticorps P, que ces protéines P1 et P2 soient elles-mêmes l'anticorps P ou la chaîne A de la ricine.

Selon un autre aspect, la présente invention concerne un procédé pour la préparation d'une immunotoxine ayant l'une des structures II ou III, dans laquelle P', W' et A' sont tels que définis ci-dessus, caractérisé en ce qu'on fait réagir en solution aqueuse et à la température ambiante une protéine de formule

P1'–($Z_1$–Y'–E')n–SH IV

avec une protéine de formule statistique

P2'–$Z_2$–Y–E–G V

où P1' et P2' représentent les radicaux des protéines $P_1$ et $P_2$ qui sont indifféremment les protéines A et P telles que définies ci-dessus, privées d'une ou plusieurs fonctions propres ou les radicaux de l'une des protéines $P_1$ ou $P_2$ provenant de l'ouverture des noyaux glucidiques des anticorps ou fragments d'anticorps par action de l'acide periodique, Y, Y', E et E' sont tels que définis ci-dessus $Z_1$ représente Z' ou NH et $Z_2$ représente Z' ou Z'' et G représente un groupe –S–S–X, où X est un groupe activateur, étant entendu que lorsque G est un groupe –S–S–X et P1' est la sous-unité A de la ricine, n est 1 ou bien n peut être zéro, mais, dans ce cas, $Z_2$ est autre qu –NH–.

Autant P que A sont donc des protéines qui présentent indifféremment:

(1) la ou les fonctions thiol qui participent au couplage et

(2) un ou plusieurs groupements fonctionnels capables de réagir avec les fonctions thiol ci-dessus pour former une liaison disulfure.

Selon la présente invention lesdites fonctions thiol et groupements fonctionnels sont ceux des protéines P ou A natives, ou bien ils y sont introduits artificiellement.

Pour des raisons de clarté on précise ci-après la signification des symboles utilisés pour désigner les protéines ci-dessus ou leurs radicaux et des expressions utilisées pour désigner les différents symboles.

Le symbole P représente une protéine choisie parmi tout anticorps ou fragment d'anticorps, ou toute immunoglobuline ou fragment d'immunoglobuline ou toute molécule dérivant des précédentes par modification artificielle de l'un quelconque de leurs groupements fonctionnels, y compris des structures glucidiques qu'elles portent, sous réserve que la protéine ainsi choisie reste capable de reconnaître sélectivement un antigène donné à la surface des cellules porteuses de cet antigène et notamment des cellules cancéreuses.

Le symbole A représente une protéine qui est la sous-unité dite chaîne A de la toxine végétale ricine telle qu'elle peut être directement obtenue à partir de la ricine naturelle ou toute molécule dérivant de cette chaîne A par modification artificielle de tout groupement fonctionnel porté par cette protéine sous réserve que la protéine ainsi choisie présente encore la propriété d'inhiber la synthèse protéique ribosomale des cellules eucaryotes telle que l'on peut la mettre en évidence dans un modèle d'étude acellulaire.

Le symbole P' représente un radical dérivé de la protéine P ci-dessus telle quelle ou chimiquement convenablement modifiée, privée d'une ou plusieurs fonctions propres et dont d'autres groupes fonctionnels sont éventuellement bloqués.

Le symbole A' représente un radical dérivé de la protéine A ci-dessus telle quelle ou chimiquement convenablement modifiée, privée d'une ou plusieurs fonctions propres et dont d'autres groupes fonctionnels sont éventuellement bloqués.

Le symbole P1 représente une des protéines A et P telles que définies ci-dessus qui porte des fonctions thiol libres, attachées à ladite protéine directement ou par l'intermédiaire d'une structure d'espacement.

Le symbole P2, différent de P1, représente une des protéines A et P telles que définies ci-dessus, qui porte un ou plusieurs groupements fonctionnels capables de réagir avec les thiols libres.

Le symbole P1' représente le radical de la protéine P1 lié aux fonctions propres de la protéine P1 notamment les fonctions SH (de la cystéine), NH2 (terminal de la protéine ou dans la position epsilon des lysines), OH (des tyrosines), COOH (des acides aspartiques et glutamiques) ou le radical de la protéine P1, provenant de l'ouverture des structures glucidiques par action de l'acide périodique lorsque P1 désigne un anticorps ou fragment d'anticorps.

Le symbole P2' représente le radical de la protéine P2 lié aux groupes fonctionnels caractéristiques NH2 (terminal de la protéine ou dans la position epsilon des lysines), OH (des tyrosines) COOH (des acides aspartiques et glutamiques).

Par exemple, P1'–SH représente la protéine P1 (qui peut être indifféremment l'anticorps ou fragment d'anticorps P ou la sous-unité A de la ricine) dans laquelle les groupes SH des cystéines sont libres et les autres groupes fonctionnels sont éventuellement bloqués.

De la même façon, P1'–CO– représente la protéine P1, dans laquelle son groupe carboxylique terminal ou les groupes carboxyliques de ses acides glutamiques et aspartiques sont couplés avec un groupement qui apporte un groupe SH introduit artificiellement.

Encore, P2'–NH– représente la protéine P2 (qui peut être indifféremment l'anticorps ou fragment d'anticorps P ou la sous-unité A de la ricine) dans laquelle son groupe amino terminal ou les groupes amino de ses lysines sont attachés à un groupement susceptible de couplage avec le thiol de la protéine P1.

Le terme «structure d'espacement inerte» tel qu'utilisé ici pour E et E' désigne un radical organique bivalent inerte vis-à-vis des réactifs utilisés dans le procédé, tel qu'un groupe alkylène à chaine droite ou ramifiée contenant de 1 à 15 atomes de carbone, qui peut contenir une ou plusieurs doubles liaisons ou qui peut être interrompu par des atomes d'oxygène ou qui peut être substitué par un ou plusieurs groupes fonctionnels inertes, tels que des groupes méthoxy, des groupes carboxyles libres ou estérifiés, des groupes dialkylamino, des groupes carbamates. Le même terme désigne également un groupe arylène contenant de 6 à 15 atomes de carbone qui peut être substitué par un ou plusieurs groupes fonctionnels inertes comme indiqué ci-dessus pour le groupe alkylène.

L'expression «groupe fonctionnel capable de se lier d'une façon covalente» telle qu'utilisée ici pour Y et Y' désigne tous les groupes susceptibles de réagir avec les fonctions propres des protéines P1 et P2 pour donner une liaison covalente. Ainsi, les groupes –CO– et –(C=NH)– sont des groupes fonctionnels convenables susceptibles de se lier aux amines libres, aux thiols et aux hydroxyles phénoliques des protéines. De même, le groupe –NH– est un groupe fonctionnel convenable susceptible de se lier aux groupes carboxyliques libres des protéines. Le groupe =N– est un groupe fonctionnel convenable, susceptible de se lier aux deux atomes de carbone des structures glucidiques des protéines P1 ou P2 après oxydation avec les ions periodate lorsque P1 ou P2 représente un anticorps ou fragment d'anticorps.

L'expression «fonction propre des protéines» telle qu'utilisée ici pour Z' et Z'', désigne les radicaux provenant des groupes caractéristiques des acides aminés formant les protéines P1 et P2, telles que l'atome d'oxygène provenant des hydroxyles des acides aminés tyrosine et éventuellement sérine, le groupe carbonyle provenant du carboxyle terminal ou des carboxyles libres des acides aspartique et glutamique, le groupe –NH– provenant de l'amine terminale des protéines ou des lysines, l'atome de soufre provenant du thiol de la cystéine. La même expression désigne également le groupe provenant de la structure dialdéhydique obtenue après oxydation d'une des structures glucidiques des protéines P1 ou P2 par traitement avec les ions periodate lorsque P1 ou P2 représente un anticorps ou fragment d'anticorps.

Le terme «radical activateur», tel qu'utilisé ici pour X, désigne un groupement lié à un pont –S–S– capable de réagir avec un thiol libre pour former un disulfure avec libération de X–SH. Des radicaux activateurs convenables sont les groupes pyridyl-2 et pyridyl-4, non substitués ou substitués par un ou des halogènes ou des radicaux alkyle, carboxyle, alkoxycarbonyle; le groupe phényle non substitué ou, de préférence, substitué par un ou des halogènes ou des groupes nitro, alkoxy, carboxyle ou alkoxycarbonyle; ou un groupe alkoxycarbonyle tel que méthoxycarbonyle.

Les termes «alkyle» et «alkoxy» désignent des groupes contenant jusqu'à 5 atomes de carbone.

Le terme «alkylène» désigne des groupements

aliphatiques saturés, à chaîne droite ou ramifiée, contenant jusqu'à 10 atomes de carbone pouvant être substitués par un ou plusieurs groupes fonctionnels inertes tels que les groupes alkoxycarbonyle.

L'obtention de la chaîne A de ricine pure, nécessaire à la réalisation des produits de la présente invention, a été décrite dans le brevet US 4 340 535. La préparation d'anticorps monoclonaux dirigés contre des cellules cancéreuses humaines a été largement mentionnée dans la littérature scientifique et beaucoup de ces anticorps sont maintenant disponibles commercialement.

Le couplage chimique entre la chaîne A de ricine et l'anticorps (ou fragment d'anticorps) peut être réalisé selon le procédé de la présente invention par des modes opératoires qui:

– préservent les activités biologiques respectives des deux composants du conjugué: l'anticorps et la chaîne A de ricine,

– assurent au procédé une reproductibilité satisfaisante et un bon rendement de couplage,

– permettent de maîtriser la valeur du rapport chaîne A de ricine/anticorps dans le conjugué obtenu,

– conduisent à un produit stable et soluble dans l'eau.

Parmi les modes opératoires répondant à ces caractéristiques, il faut privilégier ceux qui mettent en œuvre une ou plusieurs fonctions thiol pour l'établissement de la liaison entre les 2 protéines. En effet, ces fonctions thiol se prêtent particulièrement bien à l'établissement de liaisons disulfure qui satisfont aux conditions générales ci-dessus.

D'une façon générale, pour la bonne conduite des réactions de couplage entre protéines et en vue d'éliminer notamment les réticulations anarchiques, il importe que l'une des protéines à coupler et elle seule porte la ou les fonctions thiol à mettre en œuvre, alors que l'autre protéine et elle seule porte une ou plusieurs fonctions susceptibles de réagir avec les thiols en milieu aqueux de pH compris entre 5 et 9 et à une température ne dépassant pas 30 °C, pour fournir une liaison covalente stable et définie.

Les caractéristiques des protéines P1 et P2 utilisées comme produits de départ sont illustrées en détail ci-après. La structure d'espacement E peut être remplacée par les structures préférentielles R à R8 qui ne sont données qu'à titre d'exemple.

I – Cas de la protéine P1

Cette protéine étant dans tous les cas celle qui porte la ou les fonctions thiol qui participeront au couplage, la situation se présente de façon diverse selon la nature de cette protéine P1.

A) La protéine P1 porte naturellement un ou plusieurs radicaux thiol utilisables pour permettre le couplage à la protéine P2: c'est notamment le cas si la protéine P1 est le fragment d'anticorps connu sous la dénomination de F(ab)', tel qu'il est classiquement obtenu par protéolyse limitée de l'anticorps en présence de pepsine, suivie d'une réduction du (ou des) ponts disulfure entre chaînes lourdes.

C'est également le cas si la protéine P1 est la chaîne A de la ricine, ou un dérivé de cette chaîne A où l'un au moins des groupements thiol portés par les résidus de cystéine 171 et 257 de la chaîne A de ricine native est libre et accessible au couplage chimique.

Dans tous ces cas, la protéine P1 porteuse de son (ou ses) groupement(s) thiol naturel(s) peut être utilisée dans cet état pour l'étape de couplage.

B) La protéine P1 ne porte pas naturellement de radicaux thiol utilisables pour permettre le couplage à la protéine P2:

– c'est notamment le cas si la protéine P1 est une immunoglobuline native, un anticorps entier ou un fragment d'anticorps et notamment l'un des fragments couramment dénommés F(ab)'2 ou F(ab),

– un autre cas où la protéine P1 ne porte pas naturellement de fonction thiol utilisable pour le couplage est celui où cette protéine P1 est la chaîne A de ricine où chacun des deux résidus de cystéine est soit bloqué par alkylation, soit inaccessible à la modification chimique.

Dans tous les cas, il conviendra alors d'introduire artificiellement sur de telles molécules une ou plusieurs fonctions thiol aptes à permettre le couplage.

Trois types de réaction peuvent être utilisés de préférence pour l'introduction de fonctions thiol:

1 – Le premier type de réaction est l'action de l'anhydride S-acétylmercapto succinique qui est capable d'acyler des fonctions aminées de la protéine. On pourra ensuite libérer les fonctions thiol par élimination du radical acétyl protecteur, par action de l'hydroxylamine, ainsi que cela a été décrit (Archives of Biochemistry and Biophysics, 119, 41–49, 1967). On pourra même, dans le cas où la (ou les) fonction(s) thiol ainsi introduite(s) sous forme protégée doivent réagir ultérieurement avec un radical disulfure mixte activé, se dispenser de la déprotection préalable par l'hydroxylamine; en effet, la réaction de formation de la liaison disulfure utilisant les réactifs objet de la présente invention se produit aussi bien avec le radical S-acétyl qu'avec le thiol libre.

D'autres méthodes décrites dans la littérature scientifique peuvent aussi être utilisées pour l'introduction de fonctions thiol sur la protéine à modifier.

2 – Le deuxième type de réaction consiste à faire réagir la protéine par ses groupements carboxyliques avec une molécule diaminée symétrique présentant un pont disulfure, de formule:

$$H2N–R1–S–S–R1–NH2$$

dans laquelle R1 est un groupe aliphatique comportant de 2 à 5 atomes de carbone.

La réaction se fait de préférence avec la cystamine [R1 = –(CH2)2–] en présence d'un agent de couplage, tel qu'un carbodiimide et notamment un dérivé soluble dans l'eau comme

l'éthyl-1 (diméthylamino-3 propyl)-3 carbodiimide, et conduit à la formation, selon les stoechiométries mises en œuvre, de l'un des dérivés suivants ou du mélange des deux:

P1'-CO-NH-R1-S-S-R1-NH2 (Ia)
P1'-CO-NH-R1-S-S-R1-NH-CO-P1 (Ib).

Un tel produit de réaction peut être alors utilisé de deux manières: a) Si dans les formules Ia ou Ib, la protéine P1 est la chaîne A de ricine ou l'un de ses dérivés le milieu réactionnel obtenu est soumis sans fractionnement à l'action d'un réducteur, tel que le mercapto-2-éthanol, ce qui conduit à l'obtention d'un dérivé protéique unique de formule générale:

P1'-CONH-R1-SH

Le produit ainsi obtenu est alors purifié par dialyse ou filtration sur gel.

b) Si dans les formules Ia et Ib, le radical $P'_1$ est le radical de la protéine $P_1$ constitué par un anticorps ou l'un de ses fragments, le milieu réactionnel obtenu sera utilisé tel quel pour le couplage en utilisant alors une méthode d'échange thiol/disulfure par exemple celle décrite par Gilliland et Collier (Cancer Research, 40, 3564, 1980).

3 – Le troisième type de réaction consiste à utiliser des motifs glucidiques naturellement présents sur les anticorps pour fixer le radical porteur du thiol que l'on se propose d'introduire. La protéine est alors soumise à une oxydation par les ions periodate afin de faire apparaître des fonctions aldéhyde sur les motifs glucidiques. Après arrêt de la réaction par addition d'un excès d'éthylène glycol et élimination des sous-produits et excès de réactifs par dialyse, le produit obtenu est traité par une molécule diaminée symétrique présentant un pont disulfure, de formule générale:

H2N-R1-S-S-R1-NH2

dans laquelle R1 est un groupe aliphatique comportant de 2 à 5 atomes de carbone. Les produits d'addition formés sont alors réduits en amines secondaires ou tertiaires par action d'un hydrure métallique convenable, notamment le borohydrure de sodium. La réaction se fait de préférence avec la cystamine [R1 = $-(CH2)2-$] et conduit à la formation, selon les stoechiométries mises en œuvre de l'un des dérivés suivants ou du mélange des deux:

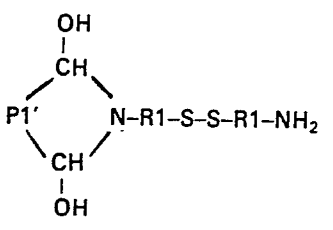

IIa

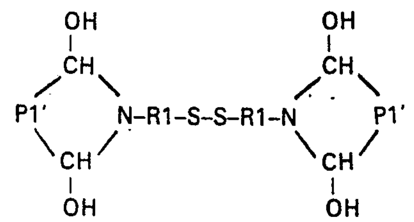

IIb

Le milieu réactionnel obtenu pourra alors être traité exactement comme indiqué ci-dessus pour les produits caractérisés par les structures Ia ou Ib, où P1' représente un anticorps ou fragment d'anticorps. Dans les deux derniers types de réaction d'introduction artificielle de groupements thiols décrits ci-dessus (ceux utilisant un réactif disulfure diaminé symétrique), il est préférable que la protéine P1 mise en œuvre ne possède ni groupes SH libres, ni groupes aminés libres.

Dans le cas de la chaîne A et de ses dérivés cela peut toujours être réalisé par alkylation du ou des SH naturels obtenue par réaction avec un réactif usuel des thiols tel que le N-éthylmaléimide ou l'acide iodacétique ou l'un de ses dérivés et par méthylation des NH2 naturels selon le procédé de méthylation réductive décrit par MEANS et FEENEY (Biochemistry 7, 2192 (1968) ). On peut introduire ainsi dans la chaîne A de ricine native jusqu'à 6 radicaux méthyle par mole. La protéine ainsi modifiée conserve l'intégralité de ses propriétés biologiques et notamment sa capacité à inhiber la synthèse protéique ribosomale dans les cellules eucaroytes.

Dans les cas des anticorps ou fragments d'anticorps et plus généralement de toutes les substances du premier groupe tel que défini précédemment qui ne possèdent pas de groupements SH naturellement libres, il conviendra de procéder à une méthylation réductive, par exemple selon la méthode de MEANS et FEENEY: on peut ainsi habituellement introduire plusieurs dizaines de radicaux méthyle par mole d'anticorps sans modifier sa capacité à reconnaître sélectivement un antigène à la surface des cellules porteuses de cet antigène.

II – Cas de la protéine P2

Cette protéine est dans tous les cas celle qui porte un ou plusieurs groupements fonctionnels capables de réagir avec les thiols de la protéine P1 pour former une liaison disulfure. Ces groupements fonctionnels, toujours artificiellement introduits sur la protéine P2, sont choisis comme indiqué ci-après.

La préparation du conjugué peut alors être représentée par le schéma:

P1'-(Z-Y-E)n-SH + P2'-Z'-Y'-E'-S-S-X

P1'-(Z-Y-E)n-S-S-E'-Y'-Z'-P2' + X-SH

La protéine P2 substituée par un atome de soufre activé est obtenue à partir de la protéine P2 elle-même ou de la protéine P2 correctement protégée, par substitution à l'aide d'un réactif lui-même

porteur d'un atome de soufre activé selon le schéma:

P2 + L–Y'–R–S–S–X      P2'–Z'–Y'–R'–S–S–X

dans lequel:

– P2 désigne la protéine à substituer

– L–Y' représente une fonction permettant la fixation covalente du réactif sur la protéine.

Le groupement fonctionnel L–Y' est une fonction capable de se lier de façon covalente avec l'une quelconque des fonctions portées par les chaînes latérales des aminoacides constitutifs de la protéine à substituer. Parmi celles-ci, on retiendra particulièrement:

a) Les fonctions aminées terminales des chaînes peptidiques ou les fonctions aminées latérales des radicaux lysyle contenus dans la protéine. Dans ce cas, L–Y' pourra notamment représenter:

– un groupe carboxylique qui pourra se lier aux fonctions aminées de la protéine en présence d'un agent de couplage tel qu'un carbodiimide et notamment un dérivé soluble dans l'eau comme l'éthyl-1 (diméthylamino-3 propyl)-3 carbodiimide;

– un chlorure d'acide carboxylique qui est susceptible de réagir directement avec les fonctions aminées pour les acyler;

– un ester dit «activé» tel qu'un ester d'ortho- ou para-, nitro- ou dinitro-phényle ou encore un ester de N-hydroxy succinimide qui peut réagir directement avec les fonctions aminées pour les acyler;

– un anhydride interne d'un diacide carboxylique tel, par exemple, l'anhydrique succinique qui réagit spontanément avec les fonctions amines pour créer des liaisons amides;

– un groupement imidoester 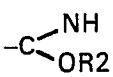

(L = OR2, Y' = –(C = NH)–)où $R_2$ est un groupe alkyle, réagissant avec les groupes aminés de la protéine P2 selon la réaction:

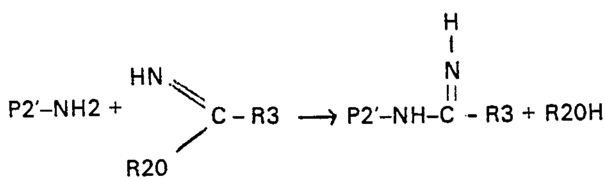

où R3 représente le groupe –R–S–SX;

b) les fonctions phé nol des radicaux tyrosyle contenus dans la protéine. Dans ce cas L–Y' pourra notamment représenter un groupement imidazolyl-1carbonyl, réagissant avec les groupes phénol de la protéine selon la réaction:

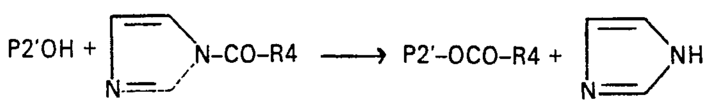

où le 1-imidazolyle est L, le groupe CO est Y' et R4 est le groupe –R–S–S–X.

Le radical –S–S–X désigne un disulfure mixte activé capable de réagir avec un radical thiol libre. En particulier, dans ce disulfure, mixte,X pourra désigner un groupe pyridyl-2 ou pyridyl-4, éventuellement substitué par un ou des radicaux alkyle, halogène ou carboxylique. X peut aussi désigner un groupe phényle de préférence substitué par un ou des groupes nitro ou carboxylique. X peut encore représenter un groupe alcoxycarbonyle, tel que le groupe méthoxycarbonyle.

Le radical R désigne la structure d'espacement (indiquée comme E dans la formule générale II ci-dessus) capable de porter simultanément les substituants Y' et S–S–X. Il devra être choisi de façon à ne pas comporter de fonctions susceptibles d'interférer au cours des réactions ultérieures avec les réactifs utilisés et les produits synthétisés. En particulier, le groupe R peut être un groupe $-(CH2)n-$ avec n compris entre 1 et 10, ou encore un groupe:

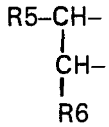

dans lequel R6 désigne l'hydrogène ou un groupe alkyle ayant de 1 à 8 atomes de carbone et R5 désigne un substituant inerte vis-à-vis des réactifs utilisés ultérieurement, tel qu'un groupe carbamate

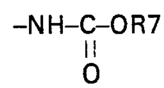

où R7 désigne un groupe alkyle droit ou ramifié ayant de 1 à 5 atomes de carbone et notamment le groupe tertiobutyle. La réaction du composé L–Y'–R–S–S–X avec la protéine P2 est effectuée en phase liquide homogène le plus souvent dans l'eau ou une solution tampon. Lorsque la solubilité des réactifs l'exige, il est possible d'ajouter au milieu réactionnel un solvant organique miscible à l'eau à une concentration finale pouvant atteindre 20% en volume lorsqu'il s'agit d'un alcool tertiaire, tel que le butanol tertiaire ou 10% en volume lorsqu'il s'agit du diméthylformamide ou du tétrahydrofuranne.

La réaction est effectuée à température ambiante pendant un temps variant de quelques minutes à quelques heures. Après quoi, une dialyse ou une filtration sur gel permet d'éliminer les produits de faible masse moléculaire et, en particulier, les excès de réactifs. Ce procédé permet d'introduire un nombre de groupements substituants par mole de protéine habituellement compris entre 1 et 15.

En utilisant de tels composés, le couplage avec la protéine P1 est réalisé par mise en présence des deux protéines en solution aqueuse de pH compris entre 6 et 8, à une température ne dépassant pas 30 °C, pendant un temps variant de 1 heure à 24 heures. La solution aqueuse obtenue est éventuellement dialysée pour éliminer les produits de faible masse moléculaire, puis le conjugué peut être purifié par diverses méthodes connues.

Parmi les composés de la présente invention, sont particulièrement convenables ceux de formule II dans laquelle w' représente un des groupements (a), (b), (c) et (d) ci-dessus, où E et E' représentent un groupe $-(CH2)p-$, p étant un nombre entier de 2 à 7 ou un groupe

$$\begin{array}{c} -CH- \\ | \\ CH2COOH \end{array}$$

Ainsi, selon un autre de ses aspects, la présente invention a pour objet des nouveaux produits ayant la formule statistique suivante

P2"–O–CO–E–G VI

dans laquelle

–P2" représente le radical d'une protéine choisie parmi:

(a) tout anticorps ou fragment d'anticorps ou toute immunoglobuline ou fragment d'immunoglobuline ou toute molécule dérivant des précédentes par modification artificielle de l'un quelconque de leurs groupements fonctionnels; et

(b) la sous-unité A de la ricine ou toute molécule dérivant de ladite sous-unité A par modification artificielle de l'un quelconque de leurs groupements fonctionnels;

ledit radical étant privé d'un ou plusieurs groupes hydroxyles phénoliques des tyrosines;

– l'atome d'oxygène est celui des groupes hydroxyles phénoliques manquant dans le radical P2";

– E et G sont tels que définis ci-dessus.

Particulièrement préférés sont les composés de formule VI dans laquelle E représente un groupe $-(CH2)p-$, où p est un nombre entier de 2 à 7 ou un groupe

$$\begin{array}{c} -CH- \\ | \\ CH2COOH \end{array}$$

et G est un groupement de structure –S–S–X, où X est un radical activateur choisi parmi les groupes 2-pyridyle et 4-pyridyle non substitués ou substitués par un ou des halogènes ou des radicaux alkyle, carboxyle, alkoxycarbonyle, le groupe phényle non substitué ou substitué par un ou des halogènes ou des groupes nitro, alkoxy, carboxyle ou alkoxycarboxyle, ou un groupe alkoxycarbonyle.

Les produits de formule VI sont préparés en faisant réagir un produit de formule:

P2"–OH

dans laquelle P2" est tel que défini ci-dessus et le groupe hydroxyle est l'hydroxyle phénolique manquant dans les tyrosines du radical P2", avec un composé de formule VII ci-dessous

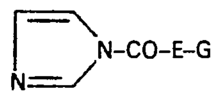
VII

à une température de 10 à 40 °C dans un solvant aqueux contenant éventuellement un solvant organique miscible à l'eau, tel que par exemple un solvant éthéré comme le dioxanne ou le tétrahydrofuranne.

Les exemples qui suivent permettent de mieux comprendre l'invention sans en limiter la portée.

Dans tous ces exemples, la préparation des conjugués sera décrite à partir de la chaîne A de ricine sous sa forme native, telle qu'elle est obtenue par le procédé décrit dans le brevet US 4 340 535.

Les anticorps utilisés dans ces exemples sont:

– soit l'anticorps monoclonal T101 dirigé contre l'antigène T65 présent sur les lymphocytes T humains et de nombreuses lignées de cellules de leucémies T humaines. Cet anticorps est celui décrit dans Journal of Immunology 125 (2), 725–7 (1980) Il est commercialement disponible auprès de HYBRITECH Inc. SAN DIEGO, California, USA

– soit l'anticorps monoclonal AT15E, dirigé contre l'antigène Thy 1.2 des lymphocytes murins. Cet anticorps est celui décrit dans Journal of Immunology 122, 2491–8 (1979) et a été obtenu à partir de l'hybridome décrit dans Hybridoma (1) 13–17 (1981)

– soit un anticorps monoclonal anti-DNP.

I – Exemple 1

P1 = chaîne A de ricine portant un SH introduit par l'intermédiaire des carboxyles.

P2 = anticorps T101 sur lequel on a introduit un groupement disulfure activé par l'intermédiaire des amines.

A) Préparation de la chaîne A de ricine correctement fonctionnalisée:

1) Blocage du thiol naturel par le N-éthylmaléimide

15 ml de solution aqueuse de chaîne A de ricine à 8 mg/ml (soit 41,1 micromoles de chaîne A) sont additionnés d'une solution aqueuse de mercapto-2-éthanol de telle sorte que la concentration finale de ce dernier soit de 1 pour cent.

La solution est laissée au repos une heure puis dialysée en continu contre du tampon phosphate 125 mM pH 7 renouvelé pendant 40 heures à raison de 300 ml/heure. Selon la méthode d'ELL-MAN (Methods in Enzymology 25, 457, 1972), on a dosé 0,9 équivalent SH par mole de chaîne A de ricine.

On procède au blocage de cette fonction SH par le N-éthylmaléïimide selon la méthode décrite dans Methods in Enzymology 11, 541, (1967). Pour cela, la chaîne A de ricine obtenue à l'étape précédente est incubée pendant 2 heures à 30 °C en présence de 20 équivalents de N-éthylmaléïimide par mole de chaîne A. On élimine l'excès de réactif par dialyse en continu contre du tampon phosphate 125 mM pH 7 renouvelé pendant 20 heures à raison de 500 ml/heure. On obtient ainsi 13 ml d'une solution de chaîne A de ricine à 7 mg/ml, ne présentant plus de groupements thiol dosables par le réactif d'ELLMAN. Le produit ainsi obtenu est désigné ultérieurement sous l'appellation chaîne A (NEM).

2) Modification des carboxyles

A 40 mg, soit 1,33 micromoles de chaîne A (NEM) dans du tampon phosphate 125 mM sont ajoutés 1,95 mmoles de chlorhydrate de cystamine et 390 microlitres d'une solution aqueuse 1 M d'éthyl-1 (diméthylamino-3 propyl)-3 carbodiimide. Le pH de la solution est ramené à 5,4 par de l'acide chlorhydrique 1 N. On laisse la réaction évoluer 2 heures à 20 °C puis elle est arrêtée par addition de 750 microlitres de solution aqueuse 1 M d'acétate de sodium et le milieu réactionnel est purifié par dialyse en continu contre du tampon phosphate 125 mM pH 7 (20 l à 300 ml/h).

Le pont disulfure de la cystamine fixée à la protéine est ensuite réduit par le mercapto-2-éthanol (à la concentration finale de 3 pour cent pendant 1 heure à 30 °C). On poursuit ensuite la dialyse contre le tampon phosphate 125 mM pH 7 comme précédemment. Après centrifugation on obtient 14 ml de solution de chaîne A (NEM) modifiée, à 1,75 mg/ml. Sur ce produit, la méthode d'ELLMAN permet de doser 0,7 groupement-SH libre par mole de protéine. Examinée par électrophorèse en gradient de polyacrylamide, cette chaîne A (NEM) modifiée présente une seule bande de poids moléculaire voisin de 30 000.

B) Préparation de l'anticorps modifié

A 25 ml d'une solution d'anticorps T101 à 10 mg/ml (soit 1,68 micromoles d'anticorps) est ajoutée une solution aqueuse contenant 11 mg d'acide (pyridyl-2 disulfanyl)-3 propionique préalablement dissous dans le butanol tertiaire et 6,5 mg d'éthyl-1 (diméthylamino-3 propyl)-3 carbodiimide. Le mélange est agité 15 minutes à 30 °C puis dialysé en continu contre du tampon phosphate 125 mM pH 7 (40 h à 500 ml/h). Après dialyse la solution protéique est centrifugée et l'on obtient 24,5 ml d'une solution à 10,3 mg d'anticorps modifié par ml. Par dosage spectrophotométrique à 343 nm de la pyridine thione-2 libérée par échange avec le mercapto-2 éthanol on constate que l'on a obtenu un anticorps portant 1,7 groupements disulfure mixte activé par mole d'anticorps.

C) Préparation de l'immunotoxine

A 1,75 ml de la solution d'anticorps activé précédemment obtenue (soit 0,12 micromole), on ajoute 7,5 ml de la solution de chaîne A (NEM) modifiée précédemment obtenue (soit 0,44 micromole) et on incube 6 heures à 30 °C. La solution est centrifugée puis purifiée par filtration sur colonne de Sephadex C100 avec mesure de la densité optique de l'effluent à 280 nm.

Le regroupement des fractions contenant à la fois l'anticorps et la chaîne A conduit à 31 ml de solution d'immunotoxine à 0,51 mg/ml (soit 15,8 mg). Cette solution contient 0,125 mg de chaîne A (NEM) modifiée couplée à l'anticorps par ml. Le taux de couplage moyen dans cette préparation est donc de 1,6 chaîne A (NEM) par mole d'anticorps.

On obtient ainsi une immunotoxine de formule II ci-dessus, où

- A' est le radical de la sous-unité A de la ricine dont les groupes SH sont bloqués par la N-éthylmaléimide
- P' est le radical de l'anticorps T101
- W' est un groupement de formule

$$-(NH-Y'-E')_n-S-S-E-Y-Z'-$$

dans laquelle

- Y' est $-CO-$
- E' est $-CH2CH2-$
- E est $-CH2CH2-$
- Y est $-NH-$
- Z' est $-CO-$
- n est 1.

D) Test d'activité des immunotoxines

La propriété biologique fondamentale de la chaîne A de ricine est d'inhiber la synthèse protéique dans les cellules eucaryotes par altération de la sous-unité ribosomale 60S.

Les tests réalisés sont donc des tests d'inhibition de la synthèse protéique:

Soit sur un modèle acellulaire (test n° 1)
soit sur un modèle cellulaire (test n° 2)

1) Le modèle acellulaire (test n° 1)

Le protocole in vitro utilise des fractions subcellulaires de foie de rat convenablement complémentées et capables d'incorporer la 14C-phénylalanine en présence d'un ARN messager artificiel: l'acide polyuridylique.

Le mode opératoire employé pour préparer les fractions subcellulaires et pour mesurer l'incorporation de 14C-phénylalanine est une adaptation de la méthode décrite dans Biochemica Biophysica Acta 312, 608–615 (1973), mettant en œuvre à la fois une fraction microsomale et une fraction de cytosol des hépatocytes de rat. L'échantillon contenant la chaîne A est introduit sous la forme d'une solution convenablement diluée dans un tampon Tris HCl 50 mM, pH 7,6 contenant du mercapto-2 éthanol à 0,2% et de la sérum albumine bovine à 15 microgrammes/ml. A partir des données de comptage, on calcule par rapport à un milieu témoin sans inhibiteur le pour-

centage d'inhibition d'incorporation de 14C-phénylalanine dans les protéines pour chaque milieu réactionnel contenant de la chaîne A de ricine. L'ensemble de ces valeurs permet de déterminer la concentration de chaîne A de ricine (ou CI50) qui inhibe 50% d'incorporation de la 14C-phenylalanine dans les conditions de l'essai.

2) Le modèle cellulaire (test n° 2)

Ce test mesure l'effet des substances étudiées sur l'incorporation de 14C-leucine dans des cellules cancéreuses en culture.

Les cellules utilisées dépendent de la spécificité de l'anticorps choisi pour la fabrication de l'immunotoxine. Dans cet exemple, il s'agit de cellules de la lignée lymphoblastoïde humaine CEM portant naturellement l'antigène T65.

Ces cellules sont incubées en présence de préparations des substances à étudier puis soumises en fin d'incubation à une mesure de leur taux d'incorporation de 14C-leucine. Cette mesure est effectuée selon une technique adaptée de la technique décrite dans Journal of Biological Chemistry 249 (11), 3557–3562 (1974) utilisant le traceur 14C-leucine pour la détermination du taux de synthèse protéique. La détermination de la radioactivité incorporée est effectuée ici sur les cellules entières isolées par filtration.

A partir de ces déterminations, on peut tracer les courbes effets/doses présentant en abscisse la concentration des substances étudiées et en ordonnée l'incorporation de 14C-leucine exprimée en pourcentage de l'incorporation par des cellules témoins en l'absence de la substance à étudier. On peut ainsi déterminer pour chaque substance étudiée la concentration qui inhibe de 50 pour cent l'incorporation de 14 C-leucine dans les cellules ou «concentration inhibitrice 50» (CI50). On a vérifié que la mesure d'incorporation de la 14C-leucine dans les cellules entières conduit à la détermination de CI50 identiques à celles obtenues par la méthode classique de mesure de la synthèse protéique.

3) Résultats

a – Test n° 1 (modèle acellulaire)

L'activité inhibitrice de la chaîne A modifiée a été déterminée. On observe une CI 50 égale à $2{,}18.10^{-10}$ mole/l. La CI50 de la chaîne A témoin de l'expérience est $1{,}03.10^{-10}$ mole/l: la modification n'entraîne donc pas de perte d'activité significative de la chaîne A.

b – Test n° 2 (modèle cellulaire)

Le test est réalisé sur cellules CEM portant naturellement l'antigène T65. Le conjugué présente une activité cytotoxique significative (CI50 environ égale à $10^{-9}$ mole/l) 50 fois supérieure à celle de la chaîne A de ricine (CI50 = 5 $10^{-8}$ mole/l dans les mèmes conditions opératoires).

En présence de chlorure d'ammonium 10 mM l'activité cytotoxique du conjugué augmente de façon très importante (CI50 = $2.10^{-12}$ mole/l). L'activité cytotoxique est alors 25 000 fois plus élevée que celle de la chaîne A.

II – Exemple 2

P1 = chaîne A de ricine sur laquelle on a introduit un groupement SH par l'intermédiaire des amines.

P2 = anticorps T101 sur lequel on a introduit un groupement disulfure activé par l'intermédiaire des amines.

A) Préparation de la chaîne A de ricine correctement fonctionnalisée.

1) Blocage au N-éthylmaléimide: identique à celui décrit à l'exemple 1.

2) Modification des amines:

A 2,5 ml de chaîne A (NEM) (0,75 micromoles) dans du tampon phosphate 125 mM pH 7 sont ajoutés 50 microlitres d'une solution d'anhydride S-acétylmercaptosuccinique à 74 mg par ml de DMF.

L'incubation dure deux heures puis le milieu est purifié de l'excès de réactif par dialyse contre du tampon phosphate 125 mM pH 7.

On obtient ainsi 2,6 ml d'une solution à 7,75 mg/ml. Par dosage spectrophotométrique des SH libérés par action de l'hydroxylamine, on constate que l'on a obtenu une chaîne A (NEM) modifiée portant 1,4 SH par mole.

B) Préparation de l'anticorps modifié: identique à celle décrite dans l'exemple 1.

C) Préparation de l'immunotoxine:

A 2,3 ml de solution d'anticorps activé précédemment obtenue (soit 0,160 micromoles), on ajoute 1,2 ml de la solution de chaîne A modifiée (soit 0,81 micromoles) en présence de 350 microlitres de solution 1 M de chlorhydrate d'hydroxylamine. L'incubation dure 5 heures à 30 °C.

Le milieu réactionnel est purifié sur une colonne de Sephadex C100 comme décrit à l'exemple 1 et on obtient 24 ml d'une solution d'immunotoxine à 0,71 mg/ml, soit 17 mg. Cette solution contient 0,130 mg de chaîne A (NEM) modifiée couplée à l'anticorps. Le taux de couplage moyen dans cette préparation est de 1,1 chaîne A (NEM) modifiée par mole d'anticorps.

On obtient ainsi une immunotoxine de formule II ci-dessus, où

– A' est le radical de la sous-unité A de la ricine dont les groupes SH sont bloqués par le N-éthylmaléimide
– P' est le radical de l'anticorps T101
– W' est un groupement de formule

$$-(NH-Y'-E')_n-S-S-E-Y-Z'-$$

dans laquelle

– Y' est –CO–
– E' est $-CH_2-CH_2-$
– E est $-\underset{|}{CH}-$ avec $CH_2-COOH$

$$E = -\underset{\displaystyle CH_2-COOH}{\underset{|}{CH}}-$$

– Y est –CO–
– Z est –NH–
– n est 1.

D) Tests d'activité:

a) Test n° 1 (modèle accellulaire)

L'activité inhibitrice de la chaîne A modifiée a été déterminée: la CI50 est égale à $1,95.10^{-10}$ mole/l. La CI50 de la chaîne A témoin de l'expérience est $1,5.10^{-10}$ mole/l. La modification n'entraîne pas de perte d'activité significative de la chaîne A.

a) Test n° 2 (modele cellulaire):

Dans les conditions expérimentales identiques à celle de l'exemple 1, la CI50, en présence de l'activateur (chlorure d'ammonium 10 mM), est de $1,6.10^{-11}$ mole/l ce qui représente une activité cytotoxique 4000 fois supérieure à celle de la chaîne A (CI50 = $2,2.10^{-7}$ mole/l). En présence de monensine (50 nM) la CI50 de cette immunotoxine est de $10^{-13}$ mole/l.

III – Exemple 3

P1 = anticorps T101 sur lequel on a introduit un SH par l'intermédiaire des amines.

P2 = Chaîne A de ricine sur laquelle on a introduit un groupement disulfure activé par l'intermédiaire de l'hydroxyle des tyrosines.

A) Préparation du réactif de couplage:

Il s'agit de l'imidazolide dérivé de l'acide (pyridyl-2 disulfanyl)-3 propionique (APDP). Cet imidazolide s'obtient en une seule étape à partir de l'APDP et du carbonyl diimidazole (CDI).

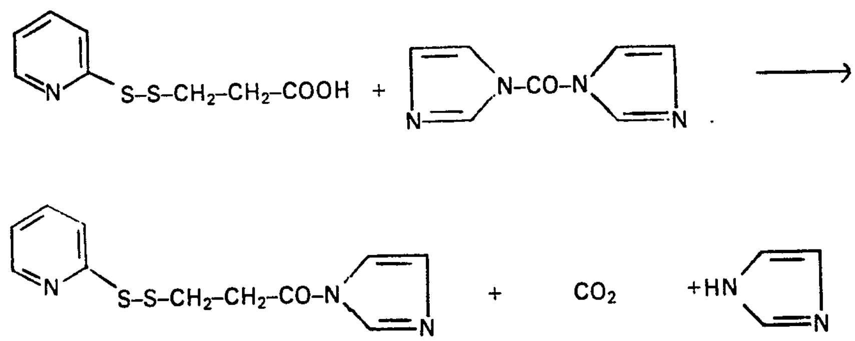

430 mg d'acide (pyridyl-2 disulfanyl)-3 propionique sont dissous dans 2 ml de THF. A cette solution sont ajoutés 405 mg de CDI. Le mélange est agité 1/4 h à 25 °C. On observe un dégagement gazeux de CO2. Le milieu réactionnel est utilisé directement et immédiatement sans purification.

B) Préparation de la chaîne A de ricine correctement fonctionnalisée

1) Blocage au N-éthylmaléimide: identique à celui décrit dans l'exemple 1.

2) Modification des tyrosines:

A 18 mg de chaîne A (NEM) dans 10 ml de tampon phosphate 125 mM pH 7 (soit 0,6 micromoles) sont ajoutés goutte à goutte 300 microlitres de la solution de réactif de couplage dans le THF obtenue précédemment. Le milieu réactionnel est agité 15 min. à 25 °C puis la solution est purifiée de l'excès de réactif par dialyse contre du tampon phosphate 125 mM pH 7. On obtient ainsi 9,5 ml d'une solution de chaîne A (NEM) modifiée à 1,55 mg/ml de protéine.

Par dosage spectrophotométrique à 343 nm de la pyridine thione-2 libérée par échange avec le mercapto-2 éthanol, on constate que l'on a obtenu une chaîne A (NEM) modifiée portant 1,6 groupements activateurs par mole de chaîne A (NEM).

C) Préparation de l'anticorps modifié:

A 45 mg d'une solution d'anticorps T101 à 9 mg/ml, soit 0,3 micromoles, sont ajoutés 25 microlitres d'une solution d'anhydride S-acétyl mercapto succinique (SAMSA) à 170 mg/ml dans le dimethylformamide. Le milieu réactionnel est agité pendant 2 heures à 4 °C puis purifié par dialyse des réactifs contre du tampon phosphate 125 mM pH 7 pendant 24 heures à un débit de 400 ml/heure. On obtient ainsi 4,1 ml d'une solution d'anticorps modifié à 8,6 mg/ml. A cette solution d'anticorps sont ajoutés 0,46 ml d'une solution de chlorhydrate d'hydroxylamine 0,5 M. Après une incubation de 1 h à 30 °C, le milieu réactionnel est purifié par dialyse des réactifs contre le tampon phosphate 125 mM pH 7.

Par dosage spectrophotométrique des groupements –SH ainsi libérés par la méthode d'ELLMAN, on constate que l'on a obtenu un anticorps portant 5 groupements activateurs par mole d'anticorps.

D) Préparation de l'immunotoxine:

A 2,1 ml de la solution d'anticorps précédemment obtenue (soit 0,11 micromole), on ajoute 5,2 ml de la solution de chaîne A modifiée (0,27 micromoles). On laisse le mélange incuber 5 heures à 30 °C.

Le milieu réactionnel est purifié sur colonne des SephadexG100 comme indiqué dans l'exemple 1. On obtient 17 ml d'une solution d'immunotoxine à 1,1 mg/ml (soit 18,7 mg). Cette solution contient 0,32 mg/ml en chaîne A (NEM) modifiée. Le taux de couplage moyen de cette préparation est de 2 chaînes A (NEM) par mole d'anticorps.

On obtient ainsi une immunotoxine de formule II ci-dessus, où

– A' est le radical de la sous-unité A de la ricine

dont les groupes SH sont bloqués par le N-éthylmaléimide

- P' est le radical de l'anticorps T101
- W' est un groupement de formule

$$-(NH-Y'-E')n-S-S-E-Y-Z'-$$

dans laquelle

- Y' est $-CO-$
- E' est $-CH(CH_2-COOH)-$
- E est $CH_2-CH_2-$
- Y est $-CO-$
- Z' est $-O-$
- n est 1.

E) Tests d'activité:

a) Test n° 1 (modèle acellulaire)

L'activité inhibitrice de la chaîne A (NEM) modifiée a été déterminée. La CI50 est égale à $3,6.10^{-10}$ mole/litre. La CI50 de la chaîne A témoin de l'expérience est de $1,2.10^{-10}$ mole/l. Il n'y a donc pas de parte d'activité significative de la chaîne A modifiée.

b) Test n° 2 (modèle cellulaire):

Dans des conditions expériementales identiques a celles de l'exemple 2, la CI50 en présence d'activateur (monensine 50 nM) est de $1,2.10^{-12}$ mole/l, ce qui représente une activité $5.10^4$ fois supérieure à celle de la chaîne A (CI50 = $6.10^{-8}$ mole/l).

IV – Exemple 4

P1 = chaîne A de ricine à l'état natif.

P2 = anticorps T101 sur lequel on a introduit un groupement disulfure activé par l'intermédiaire de l'hydroxyle des tyrosines.

A) Préparation du réactif de couplage: identique à celle décrite à l'exemple 3

B) Préparation de l'anticorps modifié

A 12,8 mg d'anticorps T101 dans 2,8 ml de tampon phosphate 125 mM pH 7, sont ajoutés goutte à goutte 34 microlitres de la solution de THF diluée au 1/2 décrite précédemment (200 équivalents/mole d'IgG). Le milieu réactionnel est agité 1/4 h à température ambiante puis purifié par dialyse. On obtient ainsi 2,6 ml d'une solution d'anticorps modifié à 4,55 mg/ml.

Par dosage spectrophotométrique à 343 nm de la pyridine thione-2 libérée par échange avec le mercapto-2 étahnol, on constate que l'on a obtenu un anticorps portant 2,2 groupements activateurs par mole d'anticorps.

C) Préparation de l'immunotoxine

A 750 microlitres d'une solution de chaîne A de ricine à 7,1 mg/ml (soit 0,177 micromoles) sont ajoutés 2,5 ml d'une solution d'anticorps activé à 4,55 mg/ml (soit 0,075 micromoles d'anticorps). On laisse le mélange incuber 18 h à 25 °C. Le milieu réactionnel est purifié sur colonne de Sephadex G100 comme décrit à l'exemple 1. On obtient 13 ml d'une solution d'immunotoxine à 0,8 mg/ml (soit 10,4 mg). Cette solution contient 0,2 mg/ml de chaîne A. Le taux de couplage moyen de cette préparation est de 1,5 chaîne A par mole d'anticorps.

On obtient ainsi une immunotoxine de formule III ci-dessus, où

- A' est le radical de la sous-unité A de la ricine
- P' est le radical de l'anticorps T101
- W' est un groupement de formule

$$-Z''-Y-E-S-S-(E'-Y'-Z')n-$$

dans laquelle

- Z'' est $-O-$
- Y est $-CO-$
- E est $-CH2-CH2-$
- n est zéro – m est 1,5

D) Tests d'activité:

- Test n° 2 (modèle cellulaire):

Dans des conditions expérimentales identiques à celles de l'exemple 1, la CI50 en présence d'activateur (Monensine 50 nM) est de $3,5.10^{-13}$ mole/l ce qui représente une activité cytotoxique $1,5.10^5$ fois supérieure à celle de la chaîne A dans la même expérience (CI50 = $0,6.10^{-8}$ mole/l).

**Revendications pour les états contractants BE, CH, DE, FR, GB, IT, LI, LU, NL, SE**

1. Une immunotoxine formée par couplage d'un anticorps P avec la sous-unité A de la ricine, ayant la formule statistique suivante

$$P'-W'-A'$$

dans laquelle P' représente le radical d'une protéine P qui est anticorps ou un fragment d'anticorps tel quel ou chimiquement convenablement modifié, privé d'une ou plusieurs fonctions propres et dans lequel d'autres groupes fonctionnels sont éventuellement bloqués, A' représente le radical d'une protéine qui est la sous-unité A de la ricine telle quelle ou chimiquement convenablement modifiée, privée d'une ou plusieurs fonctions propres et dans lequel d'autres groupes fonctionnels sont éventuellement bloqués et W' représente une structure bivalente choisie parmi

(a) un groupement de formule

$$-Z''-Y-E-S-S-(E'-Y'-Z')n-$$

(b) un groupement de formule

$$-(NH-Y'-E')n-S-S-E-Y-Z'-$$

- Z' et Z'' représentent respectivement les fonctions propres des protéines A et P, choisies parmi l'atome d'oxygène provenant de l'hydroxyle de l'un des résidus de tyrosine; le groupe carbonyle provenant de l'un des carboxyles terminaux ou de l'un des carboxyles libres des acides aspartiques et/ou glutamiques de A et P; Z'' représente aussi le groupe provenant de la structure dialdéhydique obtenue après oxydation de l'une des structures

glucidiques de P par l'acide periodique; Z' représente aussi le groupe –NH– provenant de l'une des amines terminales de A ou de l'une des amines en position epsilon de l'un des résidus de lysine de A;

– Y et Y' représentent des groupes fonctionnels capables de se lier d'une façon covalente avec l'une quelconque des fonctions Z' et Z" des protéines A et P;

– E et E' représentent des structures d'espacement inertes;

– n représente zéro ou 1.

2. Une immunotoxine formée par couplage d'un anticorps P avec la sous-unité A de la ricine, ayant la formule statistique suivante

P'(W'–A')m

dans laquelle m varie de 0,3 à 12, P' représente le radical d'une protéine P qui est un anticorps ou un fragment d'anticorps tel quel ou chimiquement convenablement modifié, privé d'une ou plusieurs fonctions propres et dans lequel les autres groupes fonctionnels sont éventuellement bloqués, A' représente le radical d'une protéine qui est la sous-unité A de la ricine telle quelle ou chimiquement convenablement modifiée, privée d'une ou plusieurs fonctions propres et dans lequel les autres groupes fonctionnels sont éventuellement bloqués et W' représente une structure bivalente choisie parmi

(a) un groupement de formule

–Z"Y–E–S–S–(E'–Y'–Z')n–

(b) un groupement de formule

–(NH–Y'–E')n–S–S–E–Y–Z'–

– Z' et Z" représentent respectivement les fonctions propres des protéines A et P, choisies parmi l'atome d'oxygène provenant de l'hydroxyle de l'un des résidus de tyrosine; le groupe carbonyle provenant de l'un des carboxyles terminaux ou de l'un des carboxyles libres des acides aspartiques et/ou glutamiques de A et P; Z" représente aussi le groupe provenant de la structure dialdéhydique obtenue après oxydation de l'une des structures glucidiques de P par l'acide periodique; Z' représente aussi le groupe –NH– provenant de l'une des amines terminales de A ou de l'une des amines en position epsilon de l'un des résidus de lysine de A;

– Y et Y' représentent des groupes fonctionnels capables de se lier d'une façon covalente avec l'une quelconque des fonctions Z' et Z" des protéines A et P;

– E et E' représentent des structures d'espacement inertes;

– n représente zéro ou 1.

3. Une immunotoxine selon la revendication 2 de formule statistique

P'(W'–A')m

dans laquelle W' et A' sont tels que définis dans la revendication 2, P' est un fragment d'anticorps Fab ou Fab' et m varie de 0,3 à 2.

4. Une immunotoxine selon la revendication 2 de formule statistique

P'(W'–A')m

dans laquelle W' et A' sont tels que définis dans la revendication 2, P' est un fragment d'anticorps F(ab')2 et m varie de 0,5 à 4.

5. Une immunotoxine selon la revendication 2 de formule statistique

P'(W'–A')m

dans laquelle W' et A' sont tels que définis dans la revendication 2, P' est un anticorps du type IgG et m varie de 0,5 à 6.

6. Une immunotoxine selon la revendication 2 de formule statistique

P'(W'–A')m

dans laquelle W' et A' sont tels que définis dans la revendication 2, P' est un anticorps du type IgM et m varie de 1 à 12.

7. Procédé pour la préparation d'une immunotoxine ayant la formule statistique suivante

P'–W'–A'

dans laquelle P' représente le radical d'un protéine P qui est un anticorps ou un fragment d'anticorps tel quel ou chimiquement convenablement modifié, privé d'une ou plusieurs fonctions propres et dans lequel les autres groupes fonctionnels sont éventuellement bloqués, A' représente le radical d'une protéine A qui est la sous-unité A de la ricine telle quelle ou chimiquement convenablement modifiée, privée d'une ou plusieurs fonctions propres et dans lequel les autres groupes fonctionnels sont éventuellement bloqués et W' représente une structure bivalente choisi parmi.

(a) un groupement de formule

–Z"–Y–E–S–S–(E'–Y'–Z')n–

(b) un groupement de formule

–(NH–Y'–E')n–S–S–E–Y–Z'–

où

– Z' et Z" représentent respectivement les fonctions propres des protéines A et P, choisies parmi l'atome d'oxygène provenant de l'hydroxyle de l'un des résidus de tyrosine; le groupe carbonyle provenant de l'un des carboxyles terminaux ou de l'un des carboxyles libres des acides aspartiques et/ou glutamiques de A et P; Z" représente aussi le groupe provenant de la structure dialdéhydique obtenue après oxydation de l'une des structures glucidiques de P par l'acide periodique; Z' représente aussi le groupe –NH– provenant de l'une des amines terminale de A ou de l'une des amines

en position epsilon de l'un des résidus de lysine de A;

– Y et Y' représentent des groupes fonctionnels capables de se lier d'une façon covalente avec l'une quelconque des fonctions Z' et Z" des protéines A et P;

– E et E' représentent des structures d'espacement inertes;

– n représente zéro ou 1, caractérisé en ce qu'on fait réagir en solution aqueuse et à la température ambiante une protéine de formule

$P1'-(Z_1-Y'-E')n-SH$ IV

avec une protéine de formule

$P2'-Z_2-Y-E-G$ V

où P1' et P2' représentent les radicaux des protéines $P_1$ et $P_2$ qui sont indifféremment les protéines A et P telles que définies dans la revendication 1, privées d'une ou plusieurs fonctions propres ou les radicaux de l'une des protéines $P_1$ ou $P_2$ provenant de l'ouverture des noyaux glucidiques des anticorps ou fragments d'anticorps par action de l'acide periodique, Y, Y', E et E' sont tels que définis ci-dessus, $Z_1$ représente Z' ou NH et $Z_2$ représente Z' ou Z" et G représente un groupe –S–S–X, où X est un groupe activateur, étant entendu que lorsque P1' est la sous unité A de la ricine, n est 1 ou bien n peut être zéro, mais, dans ce cas, $Z_2$ est autre que –NH–.

8. Procédé pour la préparation d'une immunotoxine ayant la formule statistique suivante:

$$P'(W'-A')m$$

dans laquelle m varie de 0,3 à 12, P' représente le radical d'une protéine P qui est un anticorps ou un fragment d'anticorps tel quel ou chimiquement convenablement modifié, privé d'une ou plusieurs fonctions propres et dans lequel les autres groupes fonctionnels sont éventuellement bloqués, A' représente le radical d'une protéine qui est la sous-unité A de la ricine telle quelle ou chimiquement convenablement modifiée, privée d'une ou plusieurs fonctions propres et dans lequel les autres groupes fonctionnels sont éventuellement bloqués et W' représente une structure bivalente choisie parmi

(a) un groupement de formule

$$-Z''-Y-E-S-S-(E'-Y'-Z')n-$$

(b) un groupement de formule

$$-(NH-Y'-E')n-S-S-E-Y-Z'-$$

où

– Z' et Z" représentent respectivement les fonctions propres des protéines A et P, choisies parmi l'atome d'oxygène provenant de l'hydroxyle de l'un des résidus de tyrosine; le groupe carbonyle provenant de l'un des carboxyles terminaux ou de l'un des carboxyles libres des acides aspartiques et/ou glutamiques de A et P; Z" représente aussi le groupe provenant de la structure dialdéhydique obtenue après oxydation de l'une des structures glucidiques de P par l'acide periodique; Z' représente aussi le groupe –NH– provenant de l'une des amines terminales de A ou de l'une des amines en position epsilon de l'un des résidus de lysine de A;

– Y et Y' représentent des groupes fonctionnels capables de se lier d'une façon covalente avec l'une quelconque des fonctions Z' et Z" des protéines A et P;

– E et E' représentent des structures d'espacement inertes;

– n représente zéro ou 1, caractérisé en ce qu'on fait réagir en solution aqueuse et à la température ambiante une protéine de formule

$P1'-(Z_1-Y'-E')n-SH$ IV

avec une protéine de formule

$P2'-Z_2-Y-E-G$ V

où P1' et P2' représentent les radicaux des protéines $P_1$ et $P_2$ qui sont indifféremment les protéines A et P telles que définies dans la revendication 1, privées d'une ou plusieurs fonctions propres ou les radicaux de l'une des protéines $P_1$ ou $P_2$ provenant de l'ouverture des noyaux glucidiques des anticorps ou fragments d'anticorps par action de l'acide periodique, Y, Y', E et E' sont tels que définis ci-dessus, $Z_1$ représente Z' ou NH et $Z_2$ représente Z' ou Z" et G représente un groupe –S–S–X, où X est un groupe activateur, étant entendu que lorsque P1' est la sous unité A de la ricine, n est 1 ou bien n peut être zéro, mais, dans ce cas, $Z_2$ est autre que –NH–.

9. Un produit de formule statistique

$P2''-O-CO-E-G$ VI

dans laquelle

– P2" représente le radical d'une protéine choisie parmi

(a) tout anticorps ou fragment d'anticorps ou toute immunoglobuline ou fragment d'immunoglobuline ou route molécule dérivant des précédentes par modification artificielle de l'un quelconque de leurs groupements fonctionnels; et

(b) la sous-unité A de la ricine ou toute molécule dérivant de ladite sous-unité A par modification artificielle de l'un quelconque de leurs groupements fonctionnels;

ledit radical étant privé d'un ou plusieurs groupes hydroxyles phénoliques des tyrosines

– l'atome d'oxygène est celui des groupes hydroxyles phénoliques manquant dans le radical P2";

– E représente une structure d'espacement inerte; et

– G représente un groupe

– S–S–X où X est un groupe activateur.

**Revendications pour l'état contractant AT**

1. Procédé pour la préparation d'une immunotoxine ayant la formule statistique suivante

P'–W'–A'

dans laquelle P' représente le radical d'un protéine P qui est un anticorps ou un fragment d'anticorps tel quel ou chimiquement convenablement modifié, privé d'une ou plusieurs fonctions propres et dans lequel les autres groupes fonctionnels sont éventuellement bloqués, A' représente le radical d'une protéine A qui est la sous-unité A de la ricine telle quelle ou chimiquement convenablement modifiée, privée d'une ou plusieurs fonctions propres et dans lequel les autres groupes fonctionnels sont éventuellement bloqués et W' représente une structure bivalente choisi parmi

(a) un groupement de formule

–Z'—Y–E–S–S–(E'–Y'–Z')n–

(b) un groupement de formule

–(NH–Y'–E')n–S–S–E–Y–Z'–

où

– Z' et Z'' représentent respectivement les fonctions propres des protéines A et P, choisies parmi l'atome d'oxygène provenant de l'hydroxyle de l'un des résidus de tyrosine; le groupe carbonyle provenant de l'un des carboxyles terminaux ou de l'un des carboxyles libres des acides aspartiques et/ou glutamiques de A et P; Z'' représente aussi le groupe provenant de la structure dialdéhydique obtenue après oxydation de l'une des structures glucidiques de P par l'acide periodique; Z' représente aussi le groupe –NH– provenant de l'une des amines terminale de A ou de l'une des amines en position epsilon de l'un des résidus de lysine de A;

– Y et Y' représentent des groupes fonctionnels capables de se lier d'une façon covalente avec l'une quelconque des fonctions Z' et Z'' des protéines A et P;

– E et E' représentent des structures d'espacement inertes;

– n représente zéro ou 1, caractérisé en ce qu'on fait réagir en solution aqueuse et à la température ambiante une protéine de formule

P1'–($Z_1$–Y'–E')n–SH IV

avec une protéine de formule

P2'–$Z_2$–Y–E–G V

où P1' et P2' représentent les radicaux des protéines $P_1$ et $P_2$ qui sont indifféremment les protéines A et P telles que définies dans la revendication 1, privées d'une ou plusieurs fonctions propres ou les radicaux de l'une des protéines $P_1$ ou $P_2$ provenant de l'ouverture des noyaux glucidiques des anticorps ou fragments d'anticorps par action de l'acide periodique, Y, Y', E et E' sont tels que définis ci-dessus, $Z_1$ représente Z' ou NH et $Z_2$ représente Z' ou Z'' et G représente un groupe –S–S–X, où X est un groupe activateur, étant entendu que lorsque P1' est la sous-unité A de la ricine, n est 1 ou bien n peut être zéro, mais, dans ce cas, $Z_2$ est autre que –NH–.

2. Procédé pour la préparation d'une immunotoxine ayant la formule statistique suivante

P'(W'–A')m

dans laquelle m varie de 0,3 à 12, P' représente le radical d'une protéine P qui est un anticorps ou un fragment d'anticorps tel quel ou chimiquement convenablement modifié, privé d'une ou plusieurs fonctions propres et dans lequel les autres groupes fonctionnels sont éventuellement bloqués, A' représente le radical d'une protéine qui est la sous-unité A de la ricine telle quelle ou chimiquement convenablement modifiée, privée d'une ou plusieurs fonctions propres et dans lequel les autres groupes fonctionnels sont éventuellement bloqués et W' représente une structure bivalente choisie parmi

(a) un groupement de formule

–Z''–Y–E–S–S–(E'–Y'–Z')n–

(b) un groupement de formule

–(NH–Y'–E')n–S–S–E–Y–Z'–

où

– Z' et Z'' peprésentent respectivement les fonctions propres des protéines A et P, choisies parmi l'atome d'oxygène provenant de l'hydroxyle de l'un des résidus de tyrosine; le groupe carbonyle-provenant de l'un des carboxyles terminaux ou de l'un des carboxyles libres des acides aspartiques et/ou glutamiques de A et P; Z'' représente aussi le groupe provenant de la structure dialdéhydique obtenue après oxydation de l'une des structures glucidiques de P par l'acide periodique; Z' représente aussi le groupe –NH– provenant de l'une des amines terminales de A ou de l'une des amines en position epsilon de l'un des résidus de lysine de A;

– Y et Y' représentent des groupes fonctionnels capables de se lier d'une façon covalente avec l'une quelconque des fonctions Z' et Z'' des protéines A et P;

– E et E' représentent des structures d'espacement inertes;

– n représente zéro ou 1, caractérisé en ce qu'on fait réagir en solution aqueuse et à la température ambiante une protéine de formule

P1'–($Z_1$–Y'–E')n–SH IV

avec une protéine de formule

P2'–$Z_2$–Y–E–G V

où P1' et P2' représentent les radicaux des protéines $P_1$ et $P_2$ qui sont indifféremment les protéines A et P telles que définies dans la revendication 1, privées d'une ou plusieurs fonctions propres ou les radicaux de l'une des protéines $P_1$ ou $P_2$ provenant de l'ouverture des noyaux glucidiques des anticorps ou fragments d'anticorps par action de l'acide periodique, Y, Y', E et E' sont tels que définis ci-dessus, $Z_1$ représente Z' ou NH et $Z_2$ représente Z' ou Z'' et G représente un groupe –S–S–X, où X est un groupe activateur, étant entendu que lorsque P1' est la sous unité A de la ricine, n est 1 ou bien n peut être zéro, mais, dans ce cas, $Z_2$ est autre que –NH–.

3. Procédé selon la revendication 2, pour l'obtention d'une immunotoxine de formule statistique

P'(W'–A')m

dans laquelle W' et A' sont tels que définis dans la revendication 2, P' est un fragment d'anticorps Fab ou Fab' et m varie de 0,3 à 2, caractérisé en ce que P1' représente le radical d'un fragment d'anticorps Fab ou Fab' privé d'une ou plusieurs fonctions propres ou le radical dudit fragment d'anticorps provenant de l'ouverture des noyaux glucidiques de celui-ci par l'acide périodique.

4. Procédé selon la revendication 2 pour l'obtention d'une immunotoxine de formule statistique

P'(W'–A')m

dans laquelle W' et A' sont tels que définis dans la revendication 2, P' est un fragment d'anticorps F (ab')2 et m varie de 0,5 à 4, caractérisé en ce que P1' représente le radical d'un fragment d'anticorps F(ab')2 privé d'une ou plusieurs fonctions propres ou le radical dudit fragment d'anticorps provenant de l'ouverture des noyaux glucidiques de celui-ci par l'acide périodique.

5. Procédé selon la revendication 2 pour l'obtention d'une immunotoxine de formule statistique

P'(W'–A')m

dans laquelle W' et A' sont tels que définis dans la revendication 2, P' est un anticorps du type IgG et m varie de 0,5 à 6.

6. Procédé selon la revendication 2 pour l'obtention d'une immunotoxine de formule statistique

P'(W'–A')m

dans laquelle W' et A' sont tels que définis dans la revendication 2, P' est un anticorps du type IgM et m varie de 1 à 12.

7. Procédé pour l'obtention d'un produit de formule statistique

P2''–O–CO–E–G VI

dans laquelle

– P2'' représente le radical d'une protéine choisie parmi

(a) tout anticorps ou fragment d'anticorps ou toute immunoglobine ou fragment d'immunoglobine ou toute molécule dérivant des précédentes par modification artificielle de l'un quelconque de leurs groupements fonctionnels; et

(b) la sous-unité A de la ricine ou toute molécule dérivant de ladite sous-unité A par modification artificielle de l'un quelconque de leurs groupements fonctionnels; ledit radical étant privé d'un ou plusieurs groupes hydroxyles phénoliques des tyrosines

– l'atome d'oxygène est celui des groupes hydroxyles phénoliques manquant dans le radical P2'';

– E représente une structure d'espacement inerte; et

– G représente un groupe –S–S–X où X est un groupe activateur, caractérisé en ce qu'il consiste à faire réagir un composé de formule $P_2$''–OH dans laquelle P2'' représente le radical d'une protéine choisie parmi:

(a) tout anticorps ou fragment d'anticorps ou toute immunoglobuline ou fragment d'immunoglobuline ou toute molécule dérivant des précédentes par modification artificielle de l'un quelconque de leurs groupements fonctionnels; et

(b) la sous-unité A de la ricine ou toute molécule dérivant de ladite sous-unité A par modification artificielle de l'un quelconque de leurs groupements fonctionnels;

ledit radical étant privé d'un ou plusieurs groupes hydroxyles phénoliques de tyrosines, le groupe hydroxyle est l'hydroxyle phénolique manquant dans les tyrosines du radical P2'', avec un composé de formule:

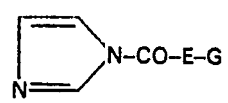

VII

dans laquelle E et G sont tels que définis ci-dessus à une température de 10 à 40 °C dans un solvant aqueux contenant éventuellement un solvant organique miscible à l'eau, tel que par exemple un solvant éthéré comme le dioxanne ou le tétrahydrofuranne.

**Patentansprüche für die Vertragsstaaten BE, CH, DE, FR, GB, IT, LI, LU, NL, SE**

1. Immunotoxin, welches durch Kopplung eines Antikörpers P mit der Untereinheit A von Ricin gebildet ist und die folgende Analysenformel hat:

P'–W'–A'

worin P' den Rest eines Proteins P darstellt, welches ein Antikörper oder ein Antikörperfragment als solches oder entsprechend chemisch modifiziert ist, dem eine oder mehrere der eigentlichen Funktionen entzogen sind und bei dem andere funktionelle Gruppen gegebenenfalls blockiert

sind, A' den Rest eines Proteins bedeutet, welches die Untereinheit A von Ricin als solche oder entsprechend chemisch modifiziert ist, der eine oder mehrere der eigentlichen Funktionen entzogen sind und bei der andere funktionelle Gruppen gegebenenfalls blockiert sind, und W' für eine bivalente Struktur steht, ausgewählt aus

(a) einer Gruppe der Formel

$$-Z''-Y-E-S-S-(E'-Y'-Z')n-$$

(b) einer Gruppe der Formel

$$-(NH-Y'-E')n-S-S-E-Y-Z'-$$

– Z' und Z'' repräsentieren die eigentlichen Funktionen der Proteine A bzw. P, ausgewählt aus dem vom Hydroxyl eines der Tyrosinreste kommenden Sauerstoffatom und der von einem der terminalen Carboxyle oder von einem der freien Carboxyle der Asparagin- und/oder Glutaminsäure von A und P kommenden Carbonylgruppe; Z'' stellt auch die Gruppe dar, die von der durch Oxidation einer der Kohlenhydratstrukturen von P mit Perjodsäure erhaltenen Dialdehydstruktur kommt; Z' bedeutet auch die von einem der terminalen Amine von A oder einem der Amine in der Position Epsilon eines der Lysinreste von A kommende Gruppe –NH–;

– Y und Y' repräsentieren funktionelle Gruppen, die sich auf kovalente Weise mit einer der Funktionen Z' und Z'' der Proteine A und P binden können;

– E und E' repräsentieren inerte Raumstrukturen;

– n steht für Null oder 1.

2. Immunotoxin, welches durch Kopplung eines Antikörpers P mit der Untereinheit A von Ricin gebildet ist und die folgende Analysenformel hat:

$$P'(W'-A')m$$

worin m von 0,3 bis 12 variiert,

P' den Rest eines Proteins P darstellt, welches ein Antikörper oder ein Antikörperfragment als solches oder entsprechend chemisch modifiziert ist, dem eine oder mehrere der eigentlichen Funktionen entzogen sind und bei dem andere funktionelle Gruppen gegebenenfalls blockiert sind, A' den Rest eines Proteins bedeutet, welches die Untereinheit A von Ricin als solche oder entsprechend chemisch modifiziert ist, der eine oder mehrere der eigentlichen Funktionen entzogen sind und bei der andere funktionelle Gruppen gegebenenfalls blockiert sind, und W' für eine bivalente Struktur steht, ausgewählt aus

(a) einer Gruppe der Formel

$$-Z''-Y-E-S-S-(E'-Y'-Z')n-$$

(b) einer Gruppe der Formel

$$-(NH-Y'-E')n-S-S-E-Y-Z'-$$

– Z' und Z'' repräsentieren die eigentlichen Funktionen der Proteine A bzw. P, ausgewählt aus dem vom Hydroxyl eines der Tyrosinreste kommenden Sauerstoffatom und der von einem der terminalen Carboxyle oder von einem der freien Carboxyle der Asparagin- und/oder Glutaminsäure von A und P kommenden Carbonylgruppe; Z'' stellt auch die Gruppe dar, die von der durch Oxidation einer der Kohlenhydratstrukturen von P mit Perjodsäure erhaltenen Dialdehydstruktur kommt; Z' bedeutet auch die von einem der terminalen Amine von A oder einem der Amine in der Position Epsilon eines der Lysinreste von A kommende Gruppe –NH–;

– Y und Y' repräsentieren funktionelle Gruppen, die sich auf kovalente Weise mit einer der Funktionen Z' und Z'' der Proteine A und P binden können;

– E und E' repräsentieren inerte Raumstrukturen;

– n steht für Null oder 1.

3. Immunotoxin nach Anspruch 2 oder Analysenformel

$$P'(W'-A')m$$

worin W' und A' wie in Anspruch 2 definiert sind, P' ein Antikörperfragment Fab oder Fab' ist und m von 0,3 bis 2 variiert.

4. Immunotoxin nach Anspruch 2 der Analysenformel

$$P'(W'-A')m$$

worin W' und A' wie in Anspruch 2 definiert sind, P' ein Antikörperfragment F(ab')2 ist und m von 0,5 bis 4 variiert.

5. Immunotoxin nach Anspruch 2 der Analysenformel

$$P'(W'-A')m$$

worin W' und A' wie in Anspruch 2 definiert sind, P' ein Antikörper des Typs IgG ist und m von 0,5 bis 6 variiert.

6. Immunotoxin nach Anspruch 2 der Analysenformel

$$P'(W'-A')m$$

worin W' und A' wie in Anspruch 2 definiert sind, P' ein Antikörper des Typs IgM ist und m von 1 bis 12 variiert.

7. Verfahren zur Herstellung eines Immunotoxins der folgenden Analysenformel:

$$P'-W'-A'$$

worin P' den Rest eines Proteins P darstellt, welches ein Antikörper oder ein Antikörperfragment als solches oder entsprechend chemisch modifiziert ist, dem eine oder mehrere der eigentlichen Funktionen entzogen sind und bei dem andere funktionelle Gruppen gegebenenfalls blockiert

sind, A' den Rest eines Proteins A bedeutet, welches die Untereinheit A von Ricin als solche oder entsprechend chemisch modifiziert ist, der eine oder mehrere der eigentlichen Funktionen entzogen sind und bei der andere funktionelle Gruppen gegebenenfalls blockiert sind, und W' für eine bivalente Struktur steht, ausgewählt aus

(a) einer Gruppe der Formel

$$-Z''-Y-E-S-S-(E'-Y'-Z')n-$$

(b) einer Gruppe der Formel

$$-(NH-Y'-E')n-S-S-E-Y-Z'-$$

worin

– Z' und Z'' die eigentlichen Funktionen der Proteine A bzw. P, ausgewählt aus dem vom Hydroxyl eines der Tyrosinreste kommenden Sauerstoffatom und der von einem der terminalen Carboxyle oder von einem der freien Carboxyle der Asparagin- und/oder Glutaminsäure von A und P kommenden Carbonylgruppe, repräsentieren;

Z'' auch die Gruppe darstellt, die von der durch Oxidation einer der Kohlenhydratstrukturen von P mit Perjodsäure erhaltenen Dialdehydstruktur kommt; Z' auch die von einem der terminalen Amine von A oder einem der Amine in der Position Epsilon eines der Lysinreste von A kommende Gruppe –NH– bedeutet;

– Y und Y' funktionelle Gruppen, die sich auf kovalente Weise mit einer der Funktionen Z' und Z'' der Proteine A und P binden können, repräsentieren;

– E und E' inerte Raumstrukturen darstellen;

– n für Null oder 1 steht, dadurch gekennzeichnet, dass man in wässeriger Lösung und bei Umgebungstemperatur ein Protein der Formel

$$P1'-(Z_1-Y'-E')n-SH \quad IV$$

mit einem Protein der Formel

$$P2'-Z_2-Y-E-G \quad V$$

worin P1' und P2' die Reste der Proteine $P_1$ und $P_2$, die ohne Unterschied die Proteine A und P, wie im Anspruch 1 definiert, sind und denen eine oder mehrere der eigentlichen Funktionen entzogen sind, oder die von der Öffnung der Kohlenhydratkerne der Antikörper oder Antikörperfragmente durch Einwirken von Perjodsäure stammenden Reste eines der Proteine $P_1$ oder $P_2$ darstellen, Y, Y', E und E' wie oben definiert sind, $Z_1$ für Z' oder NH steht und $Z_2$ für Z' oder Z'' steht und G eine Gruppe –S–S–X darstellt, worin X eine Aktivatorgruppe ist, mit der Massgabe, dass, wenn P1' die Untereinheit A von Ricin ist, n 1 bedeutet oder n auch Null sein kann, in diesem Fall aber $Z_2$ nicht –NH– i, zur Umsetzung bringt.

8. Verfahren zur Herstellung eines Immunotoxins der folgenden Analysenformel:

$$P'(W'-A')m$$

worin m von 0,3 bis 12 variiert,

P' den Rest eines Proteins P darstellt, welches ein Antikörper oder ein Antikörperfragment als solches oder entsprechend chemisch modifiziert ist, dem eine oder mehrere der eigentlichen Funktionen entzogen sind und bei dem andere funktionelle Gruppen gegebenenfalls blockiert sind, A' den Rest eines Proteins bedeutet, welches die Untereinheit A von Ricin als solche oder entsprechend chemisch modifiziert ist, der eine oder mehrere der eigentlichen Funktionen entzogen sind und bei der andere funktionelle Gruppen gegebenenfalls blockiert sind, und W' für eine bivalente Struktur steht, ausgewählt aus

(a) einer Gruppe der Formel

$$-Z''-Y-E-S-S-(E'-Y'-Z')n-$$

(b) einer Gruppe der Formel

$$-(NH-Y'-E')n-S-S-E-Y-Z'-$$

worin

– Z' und Z'' die eigentlichen Funktionen der Proteine A bzw. P, ausgewählt aus dem vom Hydroxyl eines der Tyrosinreste kommenden Sauerstoffatom und der von einem der terminalen Carboxyle oder von einem der freien Carboxyle der Asparagin- und/oder Glutaminsäure von A und P kommenden Carbonylgruppe, repräsentieren;

Z'' auch die Gruppe darstellt, die von der durch Oxidation einer der Kohlenhydratstrukturen von P mit Perjodsäure erhaltenen Dialdehydstruktur kommt; Z' auch die von einem der terminalen Amine von A oder einem der Amine in der Position Epsilon eines der Lysinreste von A kommende Gruppe –NH– bedeutet;

– Y und Y' funktionelle Gruppen, die sich auf kovalente Weise mit einer der Funktionen Z' und Z'' der Proteine A und P binden können, repräsentieren;

– E und E' inerte Raumstrukturen darstellen;

– n für Null oder 1 steht, dadurch gekennzeichnet, dass man in wässeriger Lösung und bei Umgebungstemperatur ein Protein der Formel

$$P1'-(Z_1-Y'-E')n-SH \quad IV$$

mit einem Protein der Formel

$$P2'-Z_2-Y-E-G \quad V$$

worin P1' und P2' die Reste der Proteine $P_1$ und $P_2$, die ohne Unterschied die Proteine A und P, wie im Anspruch 1 definiert, sind und denen eine oder mehrere der eigentlichen Funktionen entzogen sind, oder die von der Öffnung der Kohlenhydratkerne der Antikörper oder Antikörperfragmente durch Einwirken von Perjodsäure stammenden Reste eines der Proteine $P_1$ oder $P_2$ darstellen, Y, Y', E und E' wie oben definiert sind, $Z_1$ für Z' oder NH steht und $Z_2$ für Z' oder Z'' steht und G eine Gruppe –S–S–X darstellt, worin X eine Aktivatorgruppe ist, mit der Massgabe, dass, wenn P1' die Untereinheit A von Ricin ist, n 1 bedeutet oder n

auch Null sein kann, in diesem Fall aber $Z_2$ nicht –NH– ist, zur Umsetzung bringt.

9. Produkt der Analysenformel

P2"–O–CO–E–G VI

worin

– P2" den Rest eines Proteins, ausgewählt aus

(a) jedem Antikörper oder Antikörperfragment oder jedem Immunglobulin oder Immunglobulinfragment oder jedem durch künstliche Modifikation einer ihrer funktionellen Gruppen von den vorherigen abgeleiteten Molekül; und

(b) der Untereinheit A von Ricin oder jedem von dieser Untereinheit A durch künstliche Modifikation einer ihrer funktionellen Gruppen abgeleiteten Molekül;

darstellt, welchem Rest eine oder mehrere phenolische Hydroxylgruppe(n) der Tyrosine entzogen sind,

– das Sauerstoffatom jenes der phenolischen Hydroxylgruppen, die im Rest P2" fehlen, ist;

– E für eine inerte Raumstruktur steht; und

– G eine Gruppe

–S–S–X, worin X eine Aktivatorgruppe ist, darstellt.

**Patentansprüche für den Vertragsstaat AT**

1. Verfahren zur Herstellung eines Immunotoxins der folgenden Analysenformel:

P'–W'–A'

worin P' den Rest eines Proteins P darstellt, welches ein Antikörper oder ein Antikörperfragment als solches oder entsprechend chemisch modifiziert ist, dem eine oder mehrere der eigentlichen Funktionen entzogen sind und bei dem andere funktionelle Gruppen gegebenenfalls blockiert sind, A' den Rest eines Proteins A bedeutet, welches die Untereinheit A von Ricin als solche oder entsprechend chemisch modifiziert ist, der eine oder mehrere der eigentlichen Funktionen entzogen sind und bei der andere funktionelle Gruppen gegebenenfalls blockiert sind, und W' für eine bivalente Struktur steht, ausgewählt aus

(a) einer Gruppe der Formel

–Z"–Y–E–S–S–(E'–Y'–Z')n–

(b) einer Gruppe der Formel

–(NH–Y'–E')n–S–S–E–Y–Z'–

worin

Z' und Z" die eigentlichen Funktionen der Proteine A bzw. P, ausgewählt aus dem vom Hydroxyl eines der Tyrosinreste kommenden Sauerstoffatom und der von einem der terminalen Carboxyle oder von einem der freien Carboxyle der Asparagin- und/oder Glutaminsäure von A und P kommenden Carbonylgruppe, repräsentieren;

Z" auch die Gruppe darstellt, die von der durch Oxidation einer der Kohlenhydratstrukturen von P mit Perjodsäure erhaltenen Dialdehydstruktur kommt; Z' auch die von einem der terminalen Amine von A oder einem der Amine in der Position Epsilon eines der Lysinreste von A kommende Gruppe –NH– bedeutet;

– Y und Y' funktionelle Gruppen, die sich auf kovalente Weise mit einer der Funktionen Z' und Z" der Proteine A und P binden können, repräsentieren;

– E und E' inerte Raumstrukturen darstellen;

– n für Null oder 1 steht, dadurch gekennzeichnet, dass man in wässeriger Lösung und bei Umgebungstemperatur ein Protein der Formel

P1'–($Z_1$–Y'–E')n–SH IV

mit einem Protein der Formel

P2'–$Z_2$–Y–E–G V

worin P1' und P2' die Reste der Proteine $P_1$ und $P_2$, die ohne Unterschied die Proteine A und P, wie im Anspruch 1 definiert, sind und denen eine oder mehrere der eigentlichen Funktionen entzogen sind, oder die von der Öffnung der Kohlenhydratkerne der Antikörper oder Antikörperfragmente durch Einwirken von Perjodsäure stammenden Reste eines der Proteine $P_1$ oder $P_2$ darstellen, Y, Y', E und E' wie oben definiert sind, $Z_1$ für Z' oder NH steht und $Z_2$ für Z' oder Z" steht und G eine Gruppe –S–S–X darstellt, worin X eine Aktivatorgruppe ist, mit der Massgabe, dass, wenn P1' die Untereinheit A von Ricin ist, n 1 bedeutet oder n auch Null sein kann, in diesem Fall aber $Z_2$ nicht –NH– ist, zur Umsetzung bringt.

2. Verfahren zur Herstellung eines Immunotoxins der folgenden Analysenformel:

P'(W'–A')m

worin m von 0,3 bis 12 variiert,

P' den Rest eines Proteins P darstellt, welches ein Antikörper oder ein Antikörperfragment als solches oder entsprechend chemisch modifiziert ist, dem eine oder mehrere der eigentlichen Funktionen entzogen sind und bei dem andere funktionelle Gruppen gegebenenfalls blockiert sind, A' den Rest eines Proteins bedeutet, welches die Untereinheit A von Ricin als solche oder entsprechend chemisch modifziert ist, der eine oder mehrere der eigentlichen Funktionen entzogen sind und bei der andere funktionelle Gruppen gegebenenfalls blockiert sind, und W' für eine bivalente Struktur steht, ausgewählt aus

(a) einer Gruppe der Formel

–Z"Y–E–S–S–(E'–Y'–Z')n–

(b) einer Gruppe der Formel

–(NH–Y'–E')n– S–S–E–Y–Z'–

worin

– Z' und Z" die eigentlichen Funktionen der Proteine A bzw. P, ausgewählt aus dem vom Hydroxyl eines der Tyrosinreste kommenden Sauerstoffatom und der von einem der terminalen Carboxyle oder von einem der freien Carboxyle der Asparagin- und/oder Glutaminsäure von A und P kommenden Carbonylgruppe, repräsentieren;

Z" auch die Gruppe darstellt, die von der durch Oxidation einer der Kohlenhydratstrukturen von P mit Perjodsäure erhaltenen Dialdehydstruktur kommt; Z' auch die von einem der terminalen Amine von A oder einem der Amine in der Position Epsilon eines der Lysinreste von A kommende Gruppe –NH– bedeutet;

– Y und Y' funktionelle Gruppen, die sich auf kovalente Weise mit einer der Funktionen Z' und Z" der Proteine A und P binden können, repräsentieren;

– E und E' inerte Raumstrukturen darstellen;

– n für Null oder 1 steht, dadurch gekennzeichnet, dass man in wässeriger Lösung und bei Umgebungstemperatur ein Protein der Formel

$P1'-(Z_1-Y'-E')n-SH$ IV

mit einem Protein der Formel

$P2'-Z_2-Y-E-G$ V

worin P1' und P2' die Reste der Proteine $P_1$ und $P_2$, die ohne Unterschied die Proteine A und P, wie im Anspruch 1 definiert, sind und denen eine oder mehrere der eigentlichen Funktionen entzogen sind, oder die von der Öffnung der Kohlenhydratkerne der Antikörper oder Antikörperfragmente durch Einwirken von Perjodsäure stammenden Reste eines der Proteine $P_1$ oder $P_2$ darstellen, Y, Y', E und E' wie oben definiert sind, $Z_1$ für Z' oder NH steht und $Z_2$ für Z' oder Z" steht und G eine Gruppe –S–S–X darstellt, worin X eine Aktivatorgruppe ist, mit der Massgabe, dass, wenn P1' die Untereinheit A von Ricin ist, n 1 bedeutet oder n auch Null sein kann, in diesem Fall aber $Z_2$ nicht –NH– ist, zur Umsetzung bringt.

3. Verfahren nach Anspruch 2 zur Herstellung eines Immunotoxins der Analysenformel

P'(W'–A')m

worin W'und A' wie in Anspruch 2 definiert sind, P'ein Antikörperfragment Fab oder Fab' ist und m von 0,3 bis 2 variiert, dadurch gekennzeichnet, dass P'1 den Rest eines Antikörperfragments Fab oder Fab', dem eines oder mehrere der eigentlichen Funktionen entzogen sind, oder den Rest des Antikörperfragments, der von der Öffnung der Kohlenhydratkerne desselben mittels Perjodsäure stammt, darstellt.

4. Verfahren nach Anspruch 2 zur Herstellung eines Immunotoxins der Analysenformel

P'(W'–A')m

worin W' und A' wie in Anspruch 2 definiert sind, P' ein Antikörperfragment F (ab') 2 ist und m von 0,5 bis 4 variiert, dadurch gekennzeichnet, dass P'1 den Rest eines Antikörperfragments F (ab') 2, dem eine oder mehrere der eigentlichen Funktionen entzogen sind, oder den Rest des Antikörperfragments, der von der Öffnung der Kohlenhydratkerne desselben mittels Perjodsäure stammt, darstellt.

5. Verfahren nach Anspruch 2 zur Herstellung eines Immunotoxins der Analysenformel.

P'(W'–A')m

worin W' und A' wie in Anspruch 2 definiert sind, P' ein Antikörper des Typs IgG ist und m von 0,5 bis 6 variiert.

6. Verfahren nach Anspruch 2 zur Herstellung eines Immunotoxins der Analysenformel

P'(W'–A')m

worin W' und A' wie in Anspruch 2 definiert sind, P' ein Antikörper des Typs IgM ist und m von 1 bis 12 variiert.

7. Verfahren zur Herstellung eines Produkts der Analysenformel

P2"–O–CO–E–G VI

worin

– P2" den Rest eines Proteins, ausgewählt aus

(a) jedem Antikörper oder Antikörperfragment oder jedem Immunglobulin oder Immunglobulinfragment oder jedem durch künstliche Modifikation einer ihrer funktionellen Gruppen von den vorherigen abgeleiteten Molekül; und

(b) der Untereinheit A von Ricin oder jedem von dieser Untereinheit A durch künstliche Modifikation einer ihrer funktionellen Gruppen abgeleiteten Molekül; darstellt, welchem Rest eine oder mehrere phenolische Hydroxylgruppe(n) der Tyrosine entzogen sind,

– das Sauerstoffatom jenes der phenolischen Hydroxylgruppen, die im Rest P2" fehlen, ist;

– E für eine inerte Raumstruktur steht; und

– G eine Gruppe

–S–S–X, worin X eine Aktivatorgruppe ist, darstellt, dadurch gekennzeichnet, dass es darin besteht, dass man eine Verbindung der Formel P2"–OH, worin P2" den Rest eine Proteins, ausgewählt aus

(a) jedem Antikörper oder Antikörperfragment oder jedem Immunglobulin oder Immunglobulinfragment oder jedem durch künstliche Modifikation einer ihrer funktionellen Gruppen von den vorherigen abgeleiteten Molekül; und

(b) der Untereinheit A von Ricin oder jedem von dieser Untereinheit A durch künstliche Modifikation einer ihrer funktionellen Gruppen abgeleiteten Molekül; darstellt, welchem Rest eine oder mehrere phenolische Hydroxylgruppe(n) der Tyrosine entzogen sind, wobei die Hydroxylgruppe das in den Tyrosinen des Restes P2" fehlende phenolische Hydroxyl ist, mit einer Verbindung der Formel

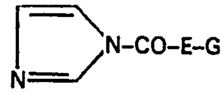 VII

worin E und G wie oben definiert sind, bei einer Temperatur von 10 bis 40 °C in einem gegebenenfalls ein mit Wasser mischbares organisches Lösungsmittel, wie beispielsweise ein etherisches Lösungsmittel, wie Dioxan oder Tetrahydrofuran, enthaltenden wässerigen Lösungsmittel zur Umsetzung bringt.

**Claims for the Contracting States BE, CH, DE, FR, GB, IT, LI, LU, NL, SE**

1. An immunotoxin formed by the coupling of an antibody P with the sub-unit A of ricin, having the following statistical formula:

$$P'-W'-A'$$

in which P' represents the radical of a protein P which is an antibody or a fragment of an antibody, as such or appropriately chemically modified, from which one or more of its own functions have been removed and in which other functional groups are optionally blocked, A' represents the radical of a protein which is the sub-unit A of ricin, as such or appropriately chemically modified, from which one or more of its own functions have been removed and in which other funtional groups are optionally blocked, and W' represents a divalent covalent structure selected from:

(a) a group of the formula:

$$-Z''-Y-E-S-S-(E'-Y'-Z')_n- \text{ or}$$

(b) a group of the formula:

$$-(NH-Y'-E')_n-S-S-E-Y-Z'-,$$

in which:

– Z' and Z' represents the functions belonging to the proteins A and P, chosen from the oxygen atom originating from the hydroxyl of one of the tyrosine residues, the carbonyl group originating from one the terminal carboxyls or the free carboxyls of the aspartic and/or glutamic acids of A and P;

Z'' also represents the group originating from the dialdehyde structure obtained after oxidation of one of the carbohydrate structures of P with periodic acid; Z' also represents the –NH– group originating from one of the terminal amines of A or from one of the amines in the epsilon position of one of the lysine residues;

– Y and Y' represent functional groups capable of bonding covalently with any one of the functions Z' and Z'' of the proteins A and P;

– E and E' represent inert spacing structures; and

– n represents zero or 1.

2. An immunotoxin formed by the coupling of an antibody P with the sub-unit A of ricin, having the following statistical formula:

$$P'(W'-A')_m$$

in which m varies between 0.3 and 12, P' represents the radical of a protein P which is an antibody or a fragment of an antibody, as such or appropriately chemically modified, from which one or more of its own functions hav been removed and in which other functional groups are optionally blocked, A' represents the radical of a protein which is the sub-unit A of ricin, as such or appropriately chemically modified, from which one or more of its own functions have been removed and in which other functional groups are optionally blocked, and W' represents a divalent structure chosen from:

(a) a group of the formula:

$$-Z''-Y-E-S-S-(E'-Y'-Z')_n-$$

(b) a group of the formula:

$$-(NH-Y'-E')_n-S-S-E-Y-Z'-,$$

– Z' and Z'' represent respectively the functions belonging to the proteins A and P, chosen from the oxygen atom originating from the hydroxyl of one of the tyrosine residues; the carbonyl group originating from one of the terminal carboxyls or from one of the free carboxyls of the aspartic and/or glutamic acids of A and P;

Z'' also represents the group originating from the dialdehyde structure obtained after oxidation of one of the carbohydrate structures of P with periodic acid, Z' also represents the –NH– group originating from one of the terminal amines of A or from of the amines in the epsilon position of one of the lysine residues of A;

– Y and Y' represent functional groups capable of bonding covalently with any one of the groups Z' and Z'' of the proteins A and P;

– E and E' represent inert spacing structures; and

– n represents zero or 1.

3. An immunotoxin according to claim 2, having the statistical formula:

$$P'(W'-A')_m$$

in which W' and A' are as defined in claim 2, P' is a fragment of antibody Fab or Fab' and m varies from 0.3 to 2.

4. An immunotoxin according to claim 2, of the statistical formula:

$$P'(W—A')_m$$

in which W' and A' are as defined in claim 2, P' is an antibody fragment $F(ab')_2$ and m varies from 0.5 to 4.

5. An immunotoxin according to claim 2, of the statistical formula:

$$P'(W'-A')_m$$

in which W' and A' are as defined in claim 2, P' is an antibody of the IgG type and m varies from 0.5 to 6.

6. An immunotoxin according to claim 2, of the statistical formula:

$$P'(W'-A')_m$$

in which W' and A' are as defined in claim 2, P' is an antibody of the IgM type and m varies from 1 to 12.

7. A process for the preparation of an immunotoxin having the following statistical formula:

$$P'-W'-A'$$

in which P' represents the radical of a protein P which is an antibody or a fragment of an antibody, as such or appropriately chemically modified, from which one or more of its own functions have been removed and in which the other functional groups are optionally blocked, A' represents the radical of a protein A which is the sub-unit A of ricin, as such or appropriately chemically modified, from which one or more of its own functions have been removed and in which the other functional groups are optionally blocked, and W' represents a divalent structure chosen from:

(a) a group of the formula:

$$-Z''-Y-E-S-S-(E'-Y'-Z')_n \text{ or}$$

(b) a group of the formula:

$$-(NH-Y'-E')_n-S-S-E-Y-Z'-,$$

in which:

– Z' and Z'' represent the functions belonging to the proteins A and P, chosen from the oxygen atom originating from the hydroxyl of one of the tyrosine residues, the carbonyl group originating from one of the terminal carboxyls or from one of the free carboxyls of the aspartic and/or glutamic acids of A and P; Z'' represents also the group originating from the dialdehyde structure obtained after oxidation of one of the carbohydrate structure of P with periodic acid; Z' represents also the –NH– group originating from one of the terminal amines of A or from one of the amines in the epsilon position of one of the A lysine residues;

– Y and Y' represent functional groups capable of bonding covalently with any one of the groups Z' and Z'' of the proteins A and P;

– E and E' represent inert spacing structures; and

– n represents zero or 1, characterized in that a protein of the formula:

$$P_1'-(Z_1-Y'-E')_n)-SH \qquad IV$$

is reacted, in aqueous solution and at ambient temperature, with a protein of the formula:

$$P_2'-Z_2-Y-E-G \qquad V$$

in which $P_1'$ and $P_2'$ represent the radicals of proteins $P_1$ and $P_2$ which are indifferently the proteins A and P as defined in claim 1, from which one or more of its own functions are removed or one of the radicals of the proteins $P_1$ or $P_2$ originating from the opening of the carbohydrate nuclei of antibodies or antibody fragments by reaction with periodic acid, Y, Y', E and E' are as defined above $Z_1$ represents Z' or NH and $Z_2$ represents Z' or Z'' and G represents a group –S–S–X, in which X is an activating group, it being understood that, when $P_1'$ is the sub-unit A of ricin, n is 1 or alternatively n can be zero, but, in this case, $Z_2$ is other than –NH–.

8. Process for the preparation of an immunotoxin having the following formula:

$$P'(W'-A')_m$$

in which m varies from 0.3 to 12, P' represents the radical of a protein P which is an antibody or a fragment of an antibody, as such or appropriately chemically modified, from which one or more of its own functions have been removed and in which the other functional groups are optionally blocked, A' represents the radical of a protein which is the sub-unit A of ricin, as such or appropriately chemically modified, from which one or more of its own functions have been removed and in which the other functional groups are optionally blocked, and W' represents a divalent structure chosen from:

(a) a group of the formula:

$$-Z''-Y-E-S-S-(E'-Y'-Z')_n- \text{ or}$$

(b) a group of the formula:

$$-(NH-Y'-E')_n-S-S-E-Y-Z'-,$$

in which:

– Z' and Z'' represent the functions belonging to the proteins A and P, chosen from the oxygen atom originating from the hydroxyl of one of the tyrosine residues, the carbonyl group originating from one of the terminal carboxyls or from one of the free carboxyls of the aspartic and/or glutamic acids of A and P; Z'' represents also the group originating from the dialdehyde structure obtained after oxidation of one of the carbohydrate structures of P with periodic acid; Z' represents also the –NH– group originating from one of the terminal amines of A or from one of the amines in the epsilon position of one of the lysine residues of A;

– Y and Y' represent functional groups capable of bonding covalently with any one of the groups, Z' and Z'' of the proteins A and P;

– E and E' represent inert spacing structures; and

– n represents zero or 1, characterized in that a protein of the formula:

$$P_1'-(Z_1-Y'-E')_n-SH \qquad IV$$

is reacted, in aqueous solution and at ambient temperature, with a protein of the formula:

$$P_2'-Z_2-Y-E-G \qquad V$$

in which $P_1'$ and $P_2'$ represent the radicals of proteins $P_1$ and $P_2$ which are indifferently the proteins A and P as defined in claim 1, from which one or more of its own functions are removed or one of the radicals of the proteins $P_1$ or $P_2$ originating from the opening of the carbohydrate nuclei of antibodies or antibody fragments by reaction with periodic acid, Y, Y', E and E' are as defined above, $Z_1$ represents Z' or NH and $Z_2$ represents Z' or Z'' and G represents a group –S–S–X, in which X is an activating group, it being understood that, when $P_1'$ is the sub-unit A of ricin, n is 1 or alternatively $P_1'$ is the sub-unit A of ricin, n is 1 or alternatively n can be zero, but, in this case, $Z_2$ is other than –NH–.

9. A product of the statistical formula:

$$P_2''\text{–O–CO–E–G} \qquad \text{VI}$$

in which:

– $P_2''$ represents the radical of a protein chosen from:

(a) any antibody or antibody fragment, any immunoglobulin or immunoglobulin fragment or any molecule derived from these molecules by artificial modification of any one of their functional groups; and

(b) the sub-unit A of ricin or any molecule derived from the said sub-unit A by artificial modification of any one of their functional groups,

one or more of the phenolic hydroxyl groups of the tyrosines having been removed from the said radical;

– the oxygen atom is that belonging to the phenolic hydroxyl groups missing from the radical $P_2''$;

– E represents an inert spacing structure; and

– G represents a group –S–S–X, in which X is an activating group.

**Claims for contracting state AT**

1. A process for the preparation of an immunotoxin having the following statistical formula:

$$P'\text{–}W'\text{–}A'$$

in which P' represents the radical of a protein P which is an antibody or a fragment of an antibody, as such or appropriately chemically modified, from which one or more of its own functions have been removed and in which the other functional groups are optionally blocked, A' represents the radical of a protein A which is the sub-unit A of ricin, as such or appropriately chemically modified, from which one or more of its own functions have been removed and in which the other functional groups are optionally blocked, and W' represents a divalent structure chosen from:

(a) a group of the formula:

$$\text{–}Z''\text{–Y–E–S–S–}(E'\text{–}Y'\text{–}Z')_n \text{ or}$$

(b) a group of the formula:

$$\text{–}(NH\text{–}Y'\text{–}E')_n\text{–S–S–E–Y–}Z'\text{–},$$

in which:

– Z' and Z'' represent the groups belonging to the proteins A and P, chosen from the oxygen atom originating from the hydroxyl of one of the tyrosine residues, the carbonyl group originating from one of the terminal carboxyls or from one of the free carboxyls of the aspartic and/or glutamic acids of A and P; Z'' represents also the group originating from the dialdehyde structure obtained after oxidation of one of the carbohydrate structure of P with periodic acid; Z' represents also the –NH– group originating from one of the terminal amines of A or from one of the amines in the epsilon position of one of the A lysine residues;

– Y and Y' represent functional groups capable of bonding covalently with any one of the functions Z' and Z'' of the proteins A and P;

– E and E' represent inert spacing structures; and

– n represents zero or 1, characterized in that a protein of formula:

$$P_1'\text{–}(Z_1\text{–}Y'\text{–}E')_n\text{–SH} \qquad \text{IV}$$

is reacted, in aqueous solution and at ambient temperature, with a protein of the formula:

$$P_2'\text{–}Z_1\text{–Y–E–G} \qquad \text{V}$$

in which $P_1'$ and $P_2'$ represent the radicals of proteins $P_1$ and $P_2$ which are indifferently the proteins A and P as defined in claim 1, from which one or more of its own functions are removed or one of the radicals of the proteins $P_1$ or $P_2$ originating from the opening of the carbohydrate nuclei of antibodies or antibody fragments by reaction with periodic acid, Y, Y', E and E' are as defined above $Z_1$ represents Z' or NH and $Z_2$ represents Z' or Z'' and G represents a group –S–S–X, in which X is an activating group, it being understood that, when $P_1'$ is the sub-unit A of ricin, n is 1 or alternatively n can be zero, but, in this case, $Z_2$ is other than –NH–.

2. Process for the preparation of an immunotoxin having the following formula:

$$P'(W'\text{–}A')_m$$

in which m varies from 0.3 to 12, P' represents the radical of a protein P which is an antibody or a fragment of an antibody, as such or appropriately chemically modified, from which one or more of its own functions have been removed and in which the other functional groups are optionally blocked, A' represents the radical of a protein which is the sub-unit A of ricin, as such or appropriately chemically modified, from which one or more of its own functions have been removed and in which the other functional groups are optionally blocked, and W' represents a divalent structure chosen from:

(a) a group of the formula:

$$\text{–}Z''\text{–Y–E–S–S–}(E'\text{–}Y'\text{–}Z')_n\text{– or}$$

(b) a group of the formula:

$$-(NH-Y'-E')_n-S-S-E-Y-Z'-,$$

in which:

– Z' and Z'' represent the functions belonging to the proteins A and P, chosen from the oxygen atom originating from the hydroxyl of one of the tyrosine residues, the carbonyl group originating from one of the terminal carboxyls or from one of the free carboxyls of the aspartic and/or glutamic acids of A and P; Z'' represents also the groups originating from the dialdehyde structure obtained after oxidation of one of the carbohydrate structures of P with periodix periodic acid; Z' represents also the –NH– group originating from one of the terminal amines of A or from one of the amines in the epsilon position of one of the lysine residues of A;

– Y and Y' represent functional groups capable of bonding covalently with any one of the groups Z' and Z'' of the proteins A and P;

– E and E' represent inert spacing structures; and

– n represents zero or 1, characterized in that a protein of the formula:

$$P_1'-(Z_1-Y'-E')_n-SH \quad IV$$

is reacted, in aqueous solution and at ambient temperatures, with a protein of the formula:

$$P_2'-Z_2-Y-E-G \quad V$$

in which $P_1'$ and $P_2'$ represent the radicals of proteins $P_1$ and $P_2$ which are indifferently the proteins A and P as defined in claim 1, from which one or more of its own functions are removed or one of the radicals of the proteins $P_1$ or $P_2$ originating from the opening of the carbohydrate nuclei of antibodies or antibody fragments by reaction with periodic acid, Y, Y', E and E' are as defined above, $Z_1$ represents Z' or NH and $Z_2$ represents Z' or Z'' and G represents a group –S–S–X, in which X is an activating group, it being understood that, when $P_1'$ is the sub-unit A of ricin, n is 1 or alternatively

n can be zero, but, in this case, $Z_2$ is other than –NH–.

3. Process according to claim 2, for the preparation of an immunotoxin having the statistical formula:

$$P'(W'-A')_m$$

in which W' and A' are as defined in claim 2, P' is an antibody fragment Fab or Fab' and m varies from 0.3 to 2, characterized in that $P_1'$ represents the radical of an antibody fragment Fab or Fab' from which one or more of its own functions have been removed or the radical of said antibody fragment originating from the opening of the carbohydrate nuclei thereof by reaction with periodic acid.

4. Process according to claim 2, for the preparation of an immunotoxin having the statistical formula

$$P'(W'-A')_m$$

in which W' and A' are as defined in claim 2, P' is an antibody fragment $F(ab')_2$ and m varies from 0.5 to 4, characterized in that $P_1'$ represents the radical of an antibody fragment $F(ab')_2$ from which one or more of its own functions have been removed or the radical of said antibody fragment originating from the opening of the carbohydrate nuclei thereof by reaction with periodic acid.

5. Process according to claim 2, for the preparation of an immunotoxin having the statistical formula

$$P'(W'-A')_m$$

in which W' and A' are as defined in claim 2, P' is an IgG type antibody and m varies from 0.5 to 6.

6. Process according to claim 2, for the preparation of an immunotoxin having the statistical formula

$$P'(W'-A')_m$$

in which W' and A' are as defined in claim 2, P' is an IgM type antibody and m varies from 1 to 12.

7. Process for the preparation of a product of the statistical formula

$$P_2''-O-CO-E-G \quad VI$$

in which

– $P_2''$ represents the radical of a protein chosen from:

(a) any antibody or antibody fragment, any immunoglobulin or immunoglobulin fragment or any molecule derived from these molecules by artificial modification of any one of their functional groups; and

(b) the sub-unit A of ricin or any molecule derived from the said sub-unit A by artificial modification of any one of their functional groups,

one or more of the phenolic hydroxyl groups of the tyrosines having been removed from the said radical;

– the oxygen atom is that belonging to the phenolic hydroxyl groups missing from the radical $P_2''$;

– E represents an inert spacing structure; and

– G represents a group –S–S–X, in which X is an activating group, characterized in that it consists in reacting a compound of formula $P_2''$–OH in which $P_2''$ represents the radical of a protein chosen from:

(a) any antibody or antibody fragment, any immunoglobulin or immunoglobulin fragment or any molecule derived from these molecules by artificial modification of any one of their functional groups; and

(b) the sub-unit A of ricin or any molecule derived from the said sub-unit A by artificial modification of any one of their functional groups;

radical from which one or more of its own phenolic hydroxyl groups of tyrosines have been removed, the hydroxyl groups is the phenolic hydroxyl missing from the tyrosines of radical $P_2''$, with a compound of formula:

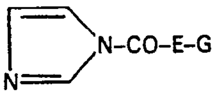

VII

in which E and G are as defined above at a temperature of 10 and 40 °C in an aqueous solvent containing optionally a water-miscible organic solvent, such as for example, an ether solvent like dioxane or tetrahydrofuran.